# EUROPEAN PATENT APPLICATION

(11) **EP 1 264 829 A1**
(43) Date of publication of application: **11.12.2002**
(21) Application number: 01908240.3
(22) Date of filing: 02.03.2001
(51) Int. Cl.: C07D 319/18, C07D 401/04, C07D 405/04, C07D 407/08, A61K 31/357, A61K 31/453, A61P 43/00, A61P 11/06, A61P 37/08, A61P 17/00, A61P 27/16, A61P 13/12, A61P 11/00, A61P 29/00, A61P 19/02, A61P 25/00, A61P 17/06

(54) **OXYGEN-CONTAINING HETEROCYCLIC COMPOUNDS**

(30) Priority: 02.03.2000 JP 2000057381
(71) Applicant: KYOWA HAKKO KOGYO CO., LTD., Chiyoda-ku, Tokyo 100-8185 (JP)
(72) Inventor: OHSHIMA, Etsuo, Sunto-gun, Shizuoka 411-8 (JP); YANAGAWA, Koji, Sunto-gun, Shizuoka 411-8 (JP); MANABE, Haruhiko, Sunto-gun, Shizuoka 411-8 (JP); MIKI, Ichiro, Sunto-gun, Shizuoka 411-8 (JP); MASUDA, Yoshiaki, Sunto-gun, Shizuoka 411-8 (JP)
(74) Representative: Casalonga, Axel
(86) International application number: JP0101611
(87) International publication number: WO01064666

(57) **Abstract**

The present invention relates to oxygen-containing heterocyclic compounds represented by the following formula (I): 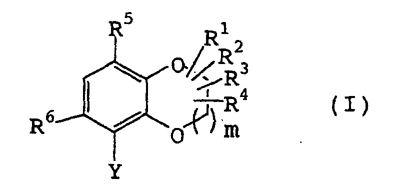 wherein m indicates an integer of 0 to 4;
R¹, R², R³ and R⁴ are the same or different, and each represents a hydrogen atom, substituted or unsubstituted lower alkyl, substituted or unsubstituted cycloalkyl, etc.; R⁵ represents hydroxy or substituted or unsubstituted lower alkoxy; R⁶ represents a hydrogen atom or halogen; and Y represents the following formula (i) or formula (ii): 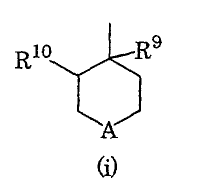 or 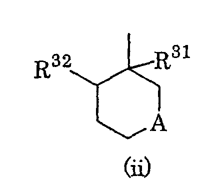 or pharmaceutically acceptable salts thereof.

## Description

### Technical Field

The present invention relates to oxygen-containing heterocyclic compounds which have a phosphodiesterase (PDE) IV inhibitory effect and are useful as a therapeutic agent for inflammatory allergic diseases such as bronchial asthma, allergic rhinitis, atopic dermatitis, nephritis, etc., autoimmune diseases such as chronic obstructive pulmonary disease, rheumatism, multiple sclerosis, Crohn's disease, psoriasis, systemic lupus erythematosus, etc., diseases in central nervous system such as depression, amnesia, dementia, etc., organ injury associated with ischemia reperfusion caused by heart failure, shock, cerebrovascular injury, etc., insulin-resistant diabetes, wound, AIDS, osteoporosis, urinary calculus, urinary incontinence, or the like.

### Background Art

It has been known that the functions of a variety of hormones and neurotransmitters are expressed by an increase in the concentration of an intracellular secondary messenger such as adenosine 3',5'-cyclic monophosphate (cAMP) or guanosine 3',5'-cyclic monophosphate (cGMP). The intracellular concentrations of cAMP and cGMP are controlled by their generation and decomposition, and the decomposition is carried out by phosphodiesterase (PDE). Therefore, the inhibition of PDE results in the increase of the concentrations of these intracellular secondary messengers. Up to the present, 8 types of PDE isozymes have been found, and it is expected that any isozyme-selective PDE inhibitors exhibit a pharmacological effect based on their physiological significance and distribution *in vivo* [Trends in Pharmacological Science, 11, p. 150 (1990); idem, 12, p. 19 (1991); and Biochemical & Biophysical Research Communications, 250, p. 751 (1998)].

It is known that the activation of the inflammatory leukocytes can be suppressed by increasing the intracellular cAMP concentration. The extraordinary activation of leukocytes induces secretion of inflammatory cytokines such as tumor necrosis factor (TNF) and expression of cellular adhesion molecules such as intercellular adhesionmolecule (ICAM) followed by cell infiltration [J. Mol. Cell. Cardiol., 12 (Suppl. II), S61 (1989)].

It is known that the contraction of a respiratory smooth muscle can be suppressed by increasing the intracellular cAMP concentration [T. J. Torphy in Directions for New Anti-Asthma Drugs, eds S.R. O'Donell et al., p. 37 (1988), Birkhauser-Verlag]. The extraordinary contraction of the respiratory smooth muscle is a main symptom of bronchial asthma. In the organ injury associated with ischemia reperfusion such as myocardial ischemia, infiltration of the inflammatory leukocytes such as neutrophils is observed in the lesion. In these inflammatory cells and respiratory smooth muscle cells, it has been elucidated that the type IV PDE (PDE IV) mainly participates in decomposition of cAMP. It is accordingly expected that PDE IV selective inhibitors would exhibit a therapeutic and/or preventive effect on inflammatory diseases, respiratory obstructive diseases, and ischemia diseases.

Further, the PDE IV inhibitors are expected to prevent the progress and spread of the inflammatory reaction transmitted by inflammatory cytokines such as TNFa and interleukin (IL)-8, since the PDE IV inhibitors suppress the secretion of these cytokines by increasing the cAMP concentration. For example, TNFα is reported to be a factor of insulin-resistant diabetes because it lowers the phosphorylation mechanism of insulin receptors in muscle and fat cells [J. Clin. Invest., 94, p. 1543 (1994). Similarly, it has been suggested that TNFα participates in the onset and progress of autoimmune diseases such as rheumatism, multiple sclerosis, Crohn's disease, etc., and accordingly PDE IV inhibitors would be effective for these diseases [Nature Medicine, 1, p. 211 (1995); idem, 1, p. 244 (1995)].

It has also been reported that TNF-α participates in tired feeling after dialysis or of cancer patients [International Journal of Artificial Organs, 21, p. 83 (1998); and Oncology Nursing Forum, 19, p. 419 (1992)]. Accordingly, PDE IV inhibitors are expected to be effective for improvement in fatigue, lassitude, etc.

A drug for increasing cAMP has been reported to accelerate the cure of wound [68th Conference of Japanese Pharmacological Society at Nagoya, Subject no. P3-116 (1995)].

It has been suggested that PDE IV inhibitors might be a therapeutic agent for osteoporosis since it shows a therapeutic effect in an animal model of osteoporosis including a cancerous osteopenia model, a sciatic nerve excised model, and an ovariectomized model [Jpn. J. Pharmacol., 79, p. 477 (1999)].

Relaxation of the ureter is known to accelerate the excretion of calculus, and so it is expected that PDE IV inhibitors could possibly be effective in treatment and/or prevention of urinary calculus since it suppresses vermicular movement of the ureter [J. Urol., 160, p.920 (1998)].

JP 7-242543 A and JP 7-242655 A disclose 1,4-benzodioxane derivatives as a therapeutic agent for liver diseases. WO 92/10494 discloses 1,4-benzodioxane derivatives having an antagonistic effect to serotonin (5HT)₃ receptors.

US 5166367 discloses 1,4-benzodioxane derivatives having an anti-hallucination effect.

JP 63-179868 A discloses 1,4-benzodioxane derivatives having a vasodilating effect.

AU 521225 discloses 1,4-benzodioxane derivatives as an intermediate for synthesis of cinnamoylpiperazines.

WO 98/22455 discloses 1,4-benzodioxane derivatives having a PDE IV inhibitory activity.

### Disclosure of the Invention

Novel and useful PDE IV inhibitors are expected to have a preventive or therapeutic effect to a wide variety of diseases. The object of the present invention is to provide oxygen-containing heterocyclic compounds which inhibit selectively PDE IV and thus increase the intracellular cAMP concentration to exhibit a bronchodilating or anti-inflammatory effect.

The present invention relates to the followings.
(1) Oxygen-containing heterocyclic compounds represented by the following formula (I): 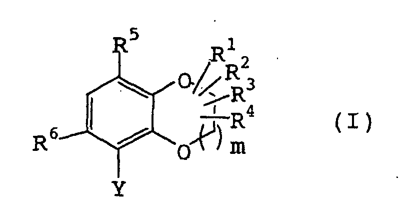 wherein m indicates an integer of 0 to 4;
   R¹, R², R³ and R⁴ are the same or different, and each represents a hydrogen atom, substituted or unsubstituted lower alkyl, substituted or unsubstituted cycloalkyl, polycycloalkyl, substituted or unsubstituted lower alkoxycarbonyl, substituted or unsubstituted lower alkanoyl, substituted or unsubstituted lower alkanoyloxy, cyano, hydroxy, substituted or unsubstituted lower alkoxy, substituted or unsubstituted lower alkenyl, substituted or unsubstituted cycloalkenyl, substituted or unsubstituted aryl, a substituted or unsubstituted aromatic heterocyclic group, substituted or unsubstituted aralkyl or -CONR⁷R⁸ (wherein R⁷ and R⁸ are the same or different, and each represents a hydrogen atom, substituted or unsubstituted lower alkyl, substituted or unsubstituted lower alkanoyl, substituted or unsubstituted cycloalkyl, substituted or unsubstituted aryl, a substituted or unsubstituted aromatic heterocyclic group or substituted or unsubstituted aralkyl, or R⁷ and R⁸ are combined together with the adjacent nitrogen atom to form a substituted or unsubstituted heterocyclic group); two groups present on the same carbon atom among R¹, R², R³ and R⁴ are combined to represent a saturated spiro carbon ring together with the said carbon atom; two groups present on the adjacent carbon atoms among R¹, R², R³ and R⁴ are combined to represent a saturated carbon ring together with the said adjacent two carbon atoms; or two groups present on the adjacent carbon atoms among R¹, R², R³ and R⁴ are combined to represent a single bond (forming a double bond together with the already-existing bond);
   R⁵ represents hydroxy or substituted or unsubstituted lower alkoxy;
   R⁶ represents a hydrogen atom or halogen; and
   Y represents the following formula (i): 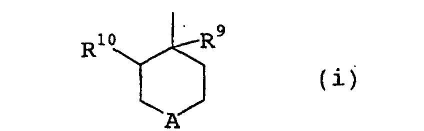 wherein A represents -CR¹¹R¹² (wherein R¹¹ and R¹² are the same or different, and each represents a hydrogen atom, hydroxy, formyl, cyano, substituted or unsubstituted lower alkoxy, a substituted or unsubstituted aromatic heterocyclic group, -NR¹³R¹⁴ (wherein R¹³ and R¹⁴ are the same or different, and each represents a hydrogen atom, substituted or unsubstituted lower alkyl, substituted or unsubstituted lower alkanoyl, substituted or unsubstituted cycloalkyl, substituted or unsubstituted aryl, a substituted or unsubstituted aromatic heterocyclic group, substituted or unsubstituted aralkyl or -SO₂R¹⁵ (wherein R¹⁵ represents lower alkyl, substituted or unsubstituted aryl or a substituted or unsubstituted aromatic heterocyclic group), or R¹³ and R¹⁴ are combined together with the adjacent nitrogen atom to form a substituted or unsubstituted heterocyclic group), -COOR¹⁶ (wherein R¹⁶ represents a hydrogen atom or substituted or unsubstituted lower alkyl), -CONR^{13a}R^{14a} (wherein R^{13a} and R^{14a} each have the same meaning as the above-described R¹³ and R¹⁴, respectively), -CONHOH or -CH₂COOR¹⁷ (wherein R¹⁷ represents a hydrogen atom or substituted or unsubstituted lower alkyl), or R¹¹ and R¹² are combined together to represent -OCH₂(CH₂)ₙO-(wherein n indicates an integer of 1 to 3), -CR¹⁸R¹⁹O- (wherein R¹⁸ and R¹⁹ are the same or different, and each represents a hydrogen atom or cyano), =CHOR²⁰ (wherein R²⁰ represents substituted or unsubstituted lower alkyl, substituted or unsubstituted lower alkenyl or substituted or unsubstituted aralkyl), =CHCOOR²¹ (wherein R²¹ represents a hydrogen atom or substituted or unsubstituted lower alkyl), =NOR²² (wherein R²² represents a hydrogen atom, substituted or unsubstituted lower alkyl, substituted or unsubstituted lower alkanoyl, substituted or unsubstituted cycloalkyl or substituted or unsubstituted aralkyl) or =O) -O-, -S(O)ₚ- (wherein p indicates an integer of 0 to 2), -COO- or -CONH-; R⁹ represents substituted lower alkyl, substituted lower alkoxy, substituted lower alkenyl, substituted or unsubstituted lower alkanoyl, substituted cycloalkyl, substituted cycloalkenyl, substituted or unsubstituted cycloalkanoyl, substituted or unsubstituted lower alkoxycarbonyl, substituted or unsubstituted aryl, a substituted or unsubstituted aromatic heterocyclic group, substituted or unsubstituted aralkyl, substituted or unsubstituted aroyl, substituted or unsubstituted heteroaroyl, -CH₂NR²³R²⁴ (wherein R²³ and R²⁴ are the same or different, and each represents a hydrogen atom, substituted or unsubstituted lower alkyl, substituted or unsubstituted lower alkanoyl, substituted or unsubstituted cycloalkyl, substituted or unsubstituted aryl, a substituted or unsubstituted aromatic heterocyclic group, substituted or unsubstituted aralkyl or -SO₂R²⁵ (wherein R²⁵ represents lower alkyl, substituted or unsubstituted aryl or a substituted or unsubstituted aromatic heterocyclic group), or R²³ and R²⁴ are combined together with the adjacent nitrogen atom to form a substituted or unsubstituted heterocyclic group), formyl, -C≡C-R²⁶ (wherein R²⁶ represents halogen, carboxy, substituted or unsubstituted lower alkoxycarbonyl, substituted or unsubstituted lower alkyl, substituted or unsubstituted lower alkanoyl, substituted or unsubstituted cycloalkyl, substituted or unsubstituted aryl, a substituted or unsubstituted aromatic heterocyclic group or substituted or unsubstituted aralkyl), -(CH₂)_{q}CONR²⁷R²⁸ (wherein q indicates an integer of 1 to 3; and R²⁷ and R²⁸ are the same or different, and each represents a hydrogen atom, substituted or unsubstituted lower alkyl, substituted or unsubstituted cycloalkyl, substituted or unsubstituted aryl, a substituted or unsubstituted aromatic heterocyclic group or substituted or unsubstituted aralkyl, or R²⁷ and R²⁸ are combined together with the adjacent nitrogen atom to form a substituted or unsubstituted heterocyclic group), -CH=CHCONR^{27a}R^{28a} (wherein R^{27a} and R^{28a} each have the same meaning as the above-defined R²⁷ and R²⁸, respectively) or -CONR²⁹R³⁰ (wherein R²⁹ and R³⁰ are the same or different, and each represents a hydrogen atom, substituted or unsubstituted lower alkyl, substituted or unsubstituted cycloalkyl, substituted or unsubstituted aryl, a substituted or unsubstituted aromatic heterocyclic group or substituted or unsubstituted aralkyl, or R²⁹ and R³⁰ are combined together with the adjacent nitrogen atom to form a substituted or unsubstituted heterocyclic group, provided that R²⁹ and R³⁰ do not simultaneously represent a hydrogen atom); R¹⁰ represents a hydrogen atom; and in the case that R¹¹ and R¹² both are a hydrogen atom at the same time, in the case that one of R¹¹ and R¹² represents cyano, -NHSO₂R¹⁵ (wherein R¹⁵ has the same meaning as defined above), -CONHOH or a substituted or unsubstituted aromatic heterocyclic group other than substituted or unsubstituted tetrazolyl among the above definition, in the case that one of R¹¹ and R¹² represents a group other than cyano, -NHSO₂R¹⁵ (wherein R¹⁵ has the same meaning as defined above), -CONHOH or a substituted or unsubstituted aromatic heterocyclic group other than substituted or unsubstituted tetrazolyl among the above definition, and the other represents a hydrogen atom or a group other than substituted or unsubstituted lower alkoxy among the above definition, or in the case that R¹¹ and R¹² are combined together to represent =NOR²² (wherein R²² has the same meaning as defined above), then R⁹ may represent cyano, ethynyl or carbamoyl, or R⁹ and R¹⁰ may be combined to represent a single bond (forming a double bond together with the already-existing bond); in the case that R¹¹ and R¹² are the same or different and do not represent a hydrogen atom, hydroxy, lower alkoxy or carboxy among the above definition, R⁹ may represent a hydrogen atom, hydroxy, lower alkyl, lower alkoxy, lower alkenyl, cycloalkyl, halogen, cycloalkenyl or carboxy, or the following formula (ii): 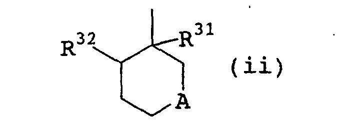 wherein A has the same meaning as defined above; R³¹ represents cyano, ethynyl, carbamoyl, substituted lower alkyl, substituted lower alkoxy, substituted lower alkenyl, substituted or unsubstituted lower alkanoyl, substituted cycloalkyl, substituted cycloalkenyl, substituted or unsubstituted cycloalkanoyl, substituted or unsubstituted lower alkoxycarbonyl, substituted or unsubstituted aryl, a substituted or unsubstituted aromatic heterocyclic group, substituted or unsubstituted aralkyl, substituted or unsubstituted aroyl, substituted or unsubstituted heteroaroyl, -CH₂NR^{23a}R^{24a} (wherein R^{23a} and R^{24a} each have the same meaning as the above-defined R²³ and R²⁴, respectively), formyl, -C≡C-R^{26a} (wherein R^{26a} has the same meaning as the above-defined R²⁶), -(CH₂)_{qa}CONR^{27b}R^{28b} (wherein qa, R^{27b} and R^{28b} each have the same meaning as the above-defined q, R²⁷ and R²⁸, respectively), -CH=CHCONR^{27c}R^{28c} (wherein R^{27c} and R^{28c} each have the same meaning as the above-defined R²⁷ and R²⁸, respectively) or -CONR^{27d}R^{28d} (wherein R^{27d} and R^{28d} each have the same meaning as the above-defined R²⁷ and R²⁸, respectively); R³² represents a hydrogen atom, or it is combined together with R³¹ to represent a single bond (forming a double bond together with the already-existing bond), and in the case that R¹¹ is not a hydrogen atom, hydroxy, lower alkoxy or carboxy among the above definition, R³¹ may represent a hydrogen atom, hydroxy, lower alkyl, lower alkoxy, lower alkenyl, cycloalkyl, halogen, cycloalkenyl or carboxy),
   or pharmaceutically acceptable salts thereof.
(2) The oxygen-containing heterocyclic compounds or pharmaceutically acceptable salts thereof according to the above item (1), wherein R⁹ is substituted lower alkyl, substituted lower alkenyl, substituted or unsubstituted cycloalkyl, substituted or unsubstituted cycloalkenyl, formyl, -C≡C-R²⁶ (wherein R²⁶ has the same meaning as defined above), -(CH₂)_{q}CONR²⁷R²⁸ (wherein q, R²⁷ and R²⁸ each have the same meaning as defined above) or -CH=CHCONR^{27a}R^{28a} (wherein R^{27a} and R^{28a} each have the same meaning as defined above), or R³¹ is substituted lower alkyl, substituted lower alkenyl, substituted or unsubstituted cycloalkyl, substituted or unsubstituted cycloalkenyl, formyl, -C≡C-R^{26a} (wherein R^{26a} has the same meaning as defined above), -(CH₂)_{qa}CONR^{27b}R^{28b} (wherein qa, R^{27b} and R^{28b} each have the same meaning as defined above) or -CH=CHCONR^{27c}R^{28c} (wherein R^{27c} and R^{28c} each have the same meaning as defined above).
(3) The oxygen-containing heterocyclic compounds or pharmaceutically acceptable salts thereof according to the above item (1) or (2), wherein R¹¹ is cyano or -COOR¹⁶ (wherein R¹⁶ has the same meaning as defined above).
(4) Pharmaceutical compositions comprising as an effective component the oxygen-containing heterocyclic compound or a pharmaceutically acceptable salt thereof according to any one of the above items (1) to (3).
(5) Phosphodiesterase IV inhibitors comprising as an effective component the oxygen-containing heterocyclic compound or a pharmaceutically acceptable salt thereof according to any one of the above items (1) to (3).
(6) Use of the oxygen-containing heterocyclic compound or a pharmaceutically acceptable salt thereof according to any one of the above items (1) to (3) for the preparation of a phosphodiesterase IV inhibitor.
(7) A method for inhibiting phosphodiesterase IV which comprises administering an effective amount of the oxygen-containing heterocyclic compound or a pharmaceutically acceptable salt thereof according to any one of the above items (1) to (3).

The compounds represented by the formula (I) are hereinafter referred to as Compound (I). Other compounds having the other formula numbers are referred to similarly.

In the definition of the groups of the formula (I), the lower alkyl moiety of the lower alkyl, the lower alkoxy, the lower alkanoyl, the lower alkanoyloxy and the lower alkoxycarbonyl includes straight-chain or branched alkyl groups having 1 to 8 carbon atoms such as methyl, ethyl, propyl, isopropyl, butyl, isobutyl, sec-butyl, tert-butyl, pentyl, hexyl, heptyl, octyl, and the like; the cycloalkyl moiety of the cycloalkyl and the cycloalkanoyl includes cycloalkyl groups having 3 to 10 carbon atoms such as cyclopropyl, cyclobutyl, cyclopentyl, cyclohexyl, cycloheptyl, cyclooctyl, cyclononyl, cyclodecyl, and the like; and the polycycloalkyl includes polycycloalkyl groups having 5 to 12 carbon atoms, such as bicyclo[3.2.1]octyl, bicyclo[4.3.2]undecyl, adamantyl, noradamantyl, and the like. The lower alkenyl includes straight-chain or branched alkenyl groups having 2 to 8 carbon atoms such as vinyl, 1-propenyl, allyl, methacryl, 1-butenyl, crotyl, pentenyl, hexenyl, heptenyl, octenyl, and the like; the cycloalkenyl includes cycloalkenyl groups having 4 to 10 carbon atoms such as cyclobutenyl, cyclopentenyl, cyclohexenyl, cycloheptenyl, cyclooctenyl, cyclononenyl, cyclodecenyl, and the like. The aryl moiety of the aryl and the aroyl includes aryl groups having 6 to 14 carbon atoms such as phenyl, naphthyl, anthryl, and the like; the aralkyl includes aralkyl groups having 7 to 15 carbon atoms such as benzyl, phenethyl, benzhydryl, naphthylmethyl, and the like. The aromatic heterocyclic group moiety of the aromatic heterocyclic group and the heteroaroyl includes 5- or 6-membered monocyclic aromatic heterocyclic groups, which contain 1 to 4 heteroatoms selected from oxygen, sulfur and nitrogen, condensed bicyclic aromatic heterocyclic groups consisting of a 5-membered ring and a 6-membered ring, and condensed bicyclic aromatic heterocyclic groups consisting of two 6-membered rings, for example, furyl, thienyl, pyridyl, pyrazinyl, pyrimidinyl, pyridazinyl, quinolyl, isoquinolyl, phthalazinyl, quinazolinyl, quinoxalinyl, naphthyridinyl, pyrrolyl, pyrazolyl, imidazolyl, triazolyl, tetrazolyl, thiazolyl, oxazolyl, isoxazolyl, oxadiazolyl, thiadiazolyl, indolyl, indazolyl, benzimidazolyl, benzotriazolyl, purinyl, phthalimido, oxathiadiazolyl, oxothiadiazolyl, oxooxadiazolyl, oxooxathiadiazolyl, thioxooxadiazolyl, and the like.

The heterocyclic group which is formed together with the adjacent nitrogen atom includes, for example, 5- or 6- or 7-membered monocyclic heterocyclic groups and condensed heterocyclic groups consisting of two 6-membered rings, specifically including pyrrolidinyl, piperidino, piperazinyl, morpholino, thiomorpholino, homopiperidino, homopiperazinyl, phthalimido, tetrahydropyridyl, tetrahydroquinolyl, tetrahydroisoquinolyl, and the like.

The saturated spiro carbon ring which is formed by two groups present on the same carbon atom together with the said carbon atom and the saturated carbon ring which is formed by two groups present on the adjacent carbon atoms together with the said two carbon atoms include those having 3 to 10 carbon atoms such as cyclopropane, cyclobutane, cyclopentane, cyclohexane, cycloheptane, cyclooctane, cyclononane, cyclodecane, and the like.

The halogen includes fluorine, chlorine, bromine and iodine atoms.

The substituents in the substituted lower alkyl, the substituted lower alkoxy, the substituted lower alkoxycarbonyl, the substituted lower alkanoyl, the substituted lower alkanoyloxy, the substituted lower alkenyl, the substituted cycloalkyl, the substituted cycloalkenyl and the substituted cycloalkanoyl are the same or different 1 to 3 substituent(s), and examples thereof include such as lower alkyl, lower alkenyl, cyano, cycloalkyl, cycloalkenyl, nitro, hydroxy, oxo, lower alkoxy, lower alkoxycarbonyl, lower alkanoyl, lower alkanoyloxy, substituted or unsubstituted aryl, a substituted or unsubstituted aromatic heterocyclic group, substituted or unsubstituted aroyl, substituted or unsubstituted heteroaroyl, carboxy, halogen, and the like. Here, the lower alkyl, the lower alkenyl, the cycloalkyl, the cycloalkenyl, the lower alkoxy, the lower alkoxycarbonyl, the lower alkanoyl, the lower alkanoyloxy, the aryl, the aromatic heterocyclic group, the aroyl, the heteroaroyl and the halogen each have the same meaning as described above. The substituents in the substituted aryl, the substituted aromatic heterocycli group, the substituted aroyl and the substituted heteroaroyl are the same or different 1 to 3 substituent(s), and examples thereof include lower alkyl, hydroxyl, lower alkoxy, lower alkanoyl, lower alkoxycarbonyl, carboxy, carbamoyl, trifluoromethyl, amino, mono- or di-lower alkyl-substituted amino, cyano, nitro, halogen, and the like. The lower alkyl moiety of the lower alkyl, the lower alkoxy, the lower alkanoyl, the lower alkoxycarbonyl and the mono- or di-lower alkyl-substituted amino and the halogen each have the same meaning as the above-described lower alkyl and halogen, respectively.

The substituents in the substituted aryl, the substituted aromatic heterocyclic group, the substituted aroyl, the substituted heteroaroyl, the substituted heterocyclic group formed together with the adjacent nitrogen atom, the substituted aralkyl and the substituted tetrazolyl are the same or different 1 to 3 substituent(s), and examples thereof include substituted or unsubstituted lower alkyl, hydroxy, oxo, lower alkoxy, lower alkanoyl, lower alkoxycarbonyl, carboxy, carbamoyl, trifluoromethyl, amino, mono- or di-lower alkyl-substituted amino, cyano, nitro, halogen, and the like. The lower alkyl moiety of the lower alkyl, the lower alkoxy, the lower alkanoyl, the lower alkoxycarbonyl, and the mono- or di-lower alkyl-substituted amino and the halogen each have the same meaning as the above-described lower alkyl and halogen, respectively. The substituent in the substituted lower alkyl has the same meaning as defined above.

The pharmaceutically acceptable salt of Compound (I) includes pharmaceutically acceptable acid addition salts, metal salts, ammonium salts, organic amine addition salts, and the like.

The pharmaceutically acceptable acid addition salt of Compound (I) includes inorganic acid salts such as hydrochlorides, sulfates, nitrates, phosphates, etc., and organic acid salts such as acetates, maleates, fumarates, citrates, etc. The pharmaceutically acceptable metal salt includes alkali metal salts such as sodium salts, potassium salts, etc., alkaline earth metal salts such as magnesium salts, calcium salts, etc., aluminum salts, zinc salts, and the like. The pharmaceutically acceptable ammonium salt includes ammonium, tetramethylammonium, and the like. The pharmaceutically acceptable organic amine addition salt includes addition salts with morpholine, piperidine, or the like.

Processes for producing Compounds (I) and their intermediates are described below.

In the following production processes, when the defined group is changed in the reaction condition or not appropriate to carry out the reaction, it is possible to obtain the desired compounds using a method for introduction and removal of protective groups conventionally used in synthetic organic chemistry [T.W. Greene and P.G.M. Wuts, Protective Groups in Organic Synthesis, 2nd Edition, John Wiley & Sons Inc., 1991]. If necessary, it is also possible to change the order of reaction steps, such as introduction of the substituent.
Production Process: Compounds (I) may be produced according to the following steps.

Compound (Ia) and Compound (Ib) may be produced according to the following reaction steps. 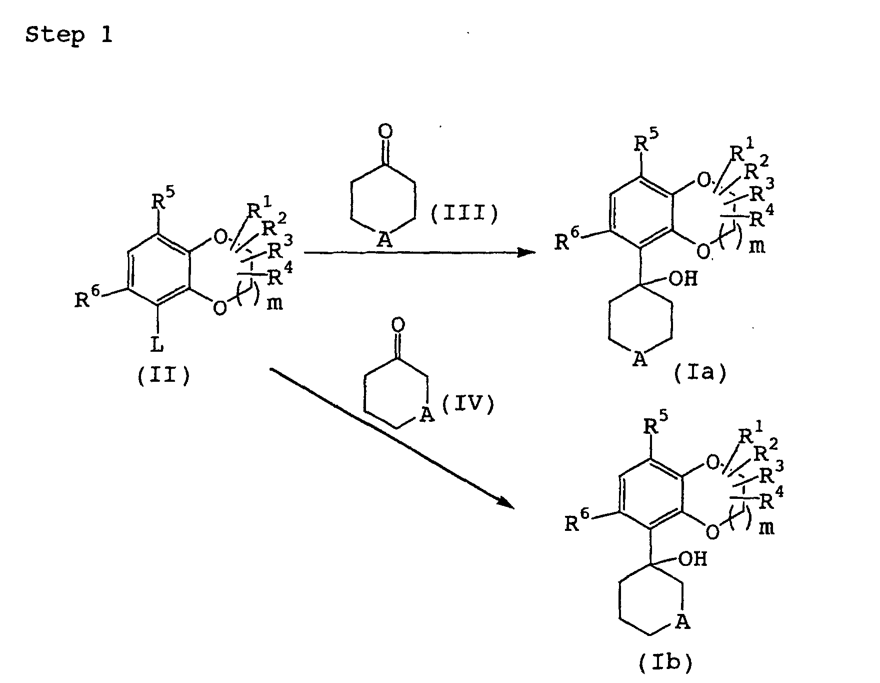 (wherein L represents chlorine, bromine or iodine; A, m, R¹, R², R³, R⁴, R⁵ and R⁶ each have the same meaning as defined above)

Compound (II) is treated with an equimolar to excess amount of a base at a temperature of -100°C to room temperature in an inert solvent for 5 minutes to 10 hours, followed by reaction with an equimolar to excess amount of Compound (III) or Compound (IV) at a temperature of -100°C to room temperature for 5 minutes to 30 hours to yield Compound (Ia) or Compound (Ib), respectively. If necessary, tetramethylethylenediamine or cerium chloride may be added.

The starting compound (II) can be obtained according to a known method (WO98/22455) or a similar method thereto. Compound (III) and Compound (IV) are commercially available.

Examples of the base are lithium, magnesium, methyllithium, methylmagnesium bromide, ethylmagnesium bromide, butyllithium, and the like.

Examples of the inert solvent are tetrahydrofuran (THF), dioxane, diethyl ether, 1,2-dimethoxyethane, diethylene glycol dimethyl ether, benzene, toluene, hexane, and the like.

Compound (Ic) and Compound (Id) can be produced according to the following reaction steps. 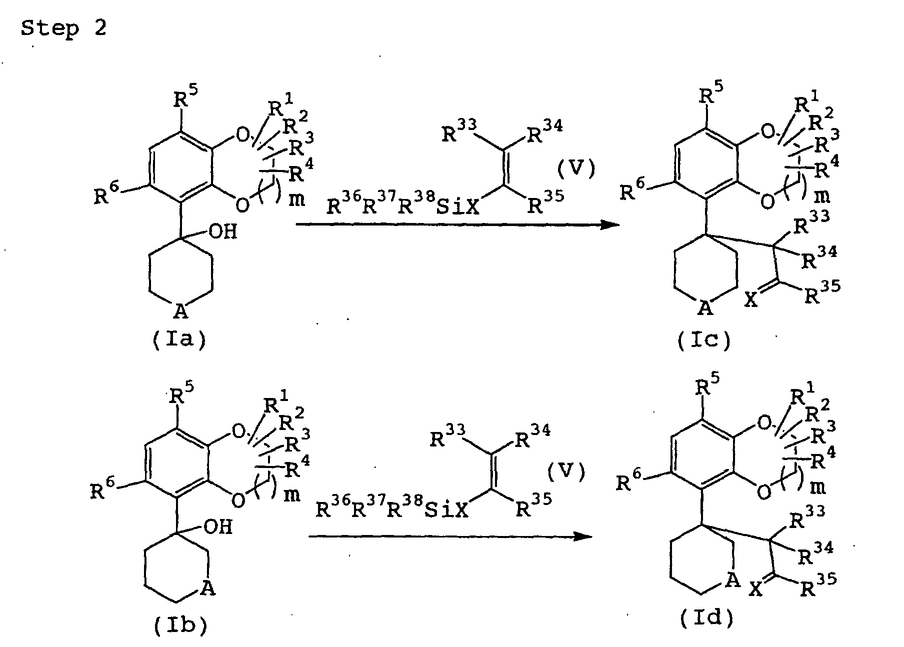 (wherein R³³, R³⁴ and R³⁵ are the same or different, and each represents a hydrogen atom, substituted or unsubstituted lower alkyl, substituted or unsubstituted cycloalkyl, substituted or unsubstituted lower alkoxycarbonyl, substituted or unsubstituted lower alkanoyl, substituted or unsubstituted lower alkanoyloxy, substituted or unsubstituted lower alkoxy, substituted or unsubstituted lower alkenyl, substituted or unsubstituted cycloalkenyl, substituted or unsubstituted aryl, a substituted or unsubstituted aromatic heterocycli group or substituted or unsubstituted aralkyl, or R³³ and R³⁵ are combined together with the adjacent two carbon atoms to form a saturated or unsaturated carbon ring; R³⁶, R³⁷ and R³⁸ are the same or different, and each represents lower alkyl or aryl; X represents O or CR⁴¹R⁴² (wherein R⁴¹ and R⁴² are the same or different, and each represents a hydrogen atom or substituted or unsubstituted lower alkyl, or one of R⁴¹ and R⁴² is combined with R³³ or R³⁵ to form a saturated or unsaturated carbon ring); and A, m, R¹, R², R³, R⁴, R⁵ and R⁶ each have the same meaning as defined above)

In the definition of R³³, R³⁴, R³⁵, R³⁶, R³⁷, R³⁸, R⁴¹ and R⁴², the substituted or unsubstituted lower alkyl, the substituted or unsubstituted cycloalkyl, the substituted or the unsubstituted lower alkoxycarbonyl, the substituted or unsubstituted lower alkanoyl, the substituted or unsubstituted lower alkanoyloxy, the substituted or unsubstituted lower alkoxy, the substituted or unsubstituted lower alkenyl, the substituted or unsubstituted cycloalkenyl, the substituted or unsubstituted aryl, the substituted or unsubstituted aromatic heterocycli group and the substituted or unsubstituted aralkyl each have the same meaning as defined above. The saturated carbon ring has the same meaning as the above-described saturated carbon ring formed together with the adjacent two carbon atoms. The unsaturated carbon ring includes those having 4 to 10 carbon atoms such as cyclobutene, cyclopentene, cyclohexene, cycloheptene, cyclooctene, cyclononene, cyclodecene, and the like.

Compound (V) is commercially available.

Compound (Ia) or Compound (Ib) is reacted with an equimolar to excess amount of Compound (V) in an inert solvent in the presence of an equimolar to excess amount of an acid at a temperature of -100°C to the boiling point of the solvent used for 5 minutes to 48 hours to yield Compound (Ic) or Compound (Id).

Examples of the acid are hydrochloric acid, sulfuric acid, 10-camphorsulfonic acid, acetic acid, formic acid, trifluoroacetic acid, p-toluenesulfonic acid, methanesulfonic acid, titanium tetrachloride, boron trifluoride, aluminum chloride, and the like.

Examples of the inert solvent are THF, dioxane, diethyl ether, 1,2-dimethoxyethane, methanol, ethanol, acetonitrile, dichloromethane, 1,2-dichloroethane, chloroform, toluene, and the like.

Compound (Ig) and Compound (Ih) can be produced according to the following reaction steps. 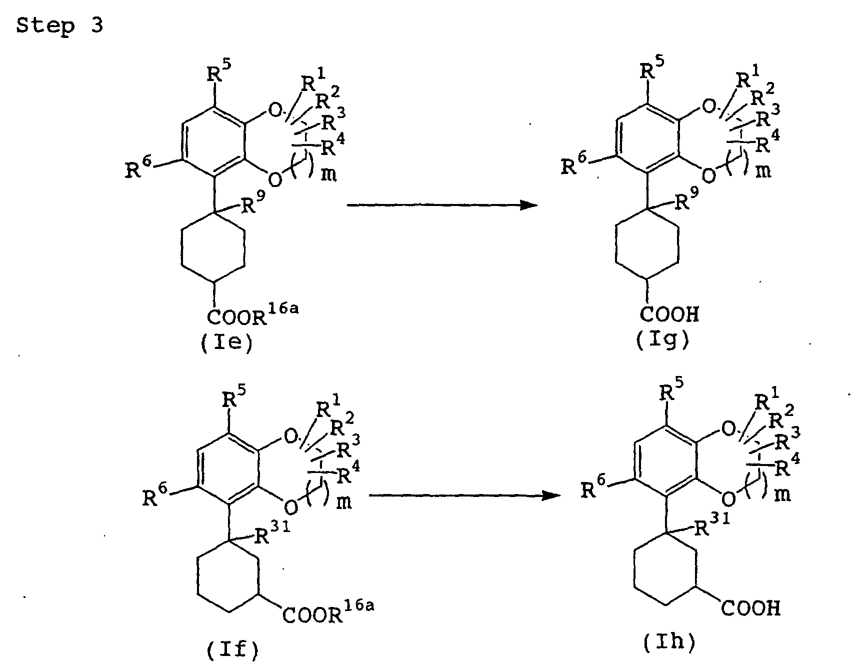 (wherein m, R¹, R², R³, R⁴, R⁵, R⁶, R⁹ and R³¹ each have the same meaning as defined above; and R^{16a} represents the substituted or unsubstituted lower alkyl as defined above)

Compound (Ie) or Compound (If) prepared according to the method of the step 2 or a similar method thereto is treated with an aqueous alkaline solution or a catalytic amount to excess amount of an acid in or without an inert solvent at a temperature of 0°C to the boiling point of the solvent used for 5 minutes to 48 hours to yield Compound (Ig) or (Ih), respectively.

Examples of the aqueous alkaline solution are aqueous solutions of sodium hydroxide, potassium hydroxide, lithium hydroxide, or the like. Examples of the acid are hydrochloric acid, sulfuric acid, 10-camphorsulfonic acid, acetic acid, formic acid, trifluoroacetic acid, p-toluenesulfonic acid, methanesulfonic acid, titanium tetrachloride, boron trifluoride, aluminum chloride, and the like.

Examples of the inert solvent are ethanol, dioxane, acetone, 2-butanone, methanol, THF, diethyl ether, 1,2-dimethoxyethane, methanol, ethanol, acetonitrile, dichloromethane, 1,2-dichloroethane, chloroform, toluene, a mixed solvent of ethanol/THF, a mixed solvent of methanol/THF, dimethylsulfoxide (DMSO), and the like.

Compound (Ik) and Compound (Im) can be produced according to the following reaction steps. 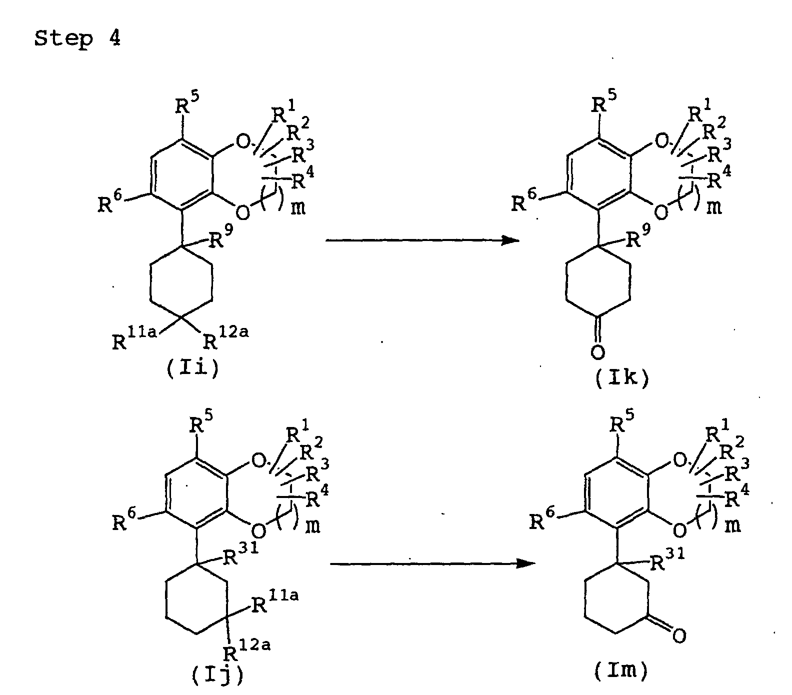 (wherein m, R¹, R², R³, R⁴, R⁵, R⁶, R⁹ and R³¹ each have the same meaning as defined above; and R^{11a} and R^{12a} are the same or different, and each represents the substituted or unsubstituted lower alkoxy as defined above, or they are combined together to represent -OCH₂(CH₂)ₙO- (wherein n has the same meaning as defined above))

Compound (Ii) or Compound (Ij) is treated with a catalytic amount to excess amount of an acid in or without an inert solvent at a temperature of 0°C to the boiling point of the solvent used for 5 minutes to 48 hours to yield Compound (Ik) or (Im), respectively.

Examples of the acid are hydrochloric acid, sulfuric acid, 10-camphorsulfonic acid, acetic acid, formic acid, trifluoroacetic acid, p-toluenesulfonic acid, methanesulfonic acid, titanium tetrachloride, boron trifluoride, aluminum chloride, and the like.

Examples of the inert solvent are THF, dioxane, diethyl ether, 1,2-dimethoxyethane, methanol, ethanol, acetone, 2-butanone, acetonitrile, dichloromethane, 1,2-dichloroethane, chloroform, toluene, and the like.

The compounds in which R⁹ or R³¹ is a hydrogen atom, cyano, substituted or unsubstituted lower alkoxy, substituted or unsubstituted lower alkyl, substituted or unsubstituted lower alkenyl, substituted or unsubstituted cycloalkyl, substituted or unsubstituted cycloalkenyl, substituted or unsubstituted aryl, a substituted or unsubstituted aromatic heterocyclic group or substituted or unsubstituted aralkyl may be produced in a method similar to that in the step 2 using a reducing agent such as triethylsilane, sodium borohydride, or the like, a cyanide such as trimethylsilyl cyanide, sodium cyanide, or the like, an alcohol, a trialkylaluminum, a tetraalkyltitanium, a lithium compound of alkyl, alkenyl, cycloalkyl, cycloalkenyl, aryl, an aromatic heterocyclic group or aralkyl, or an organometallic reagent such as magnesium chloride, magnesium bromide, magnesium iodide, or the like, instead of Compound (V).

The compounds in which R⁹ or R³¹ is carboxy, carbamoyl, formyl, substituted or unsubstituted lower alkanoyl, substituted or unsubstituted alkoxycarbonyl, substituted or unsubstituted cycloalkanoyl, substituted or unsubstituted aroyl or substituted or unsubstituted heteroaroyl may be produced from the compounds in which R⁹ or R³¹ is cyano in a known method [Jikken Kagaku Kohza (Manual for Experimental Chemistry), 4th edition, Edited by Chemical Society of Japan, vol. 22, pp. 12-13 and pp. 151-154 (1991); idem, vol. 21, pp. 89-94 and pp. 289-290 (1991)] or a similar method thereto.

The compounds in which R⁹ is ethynyl or -C≡C-R²⁶ (wherein R²⁶ has the same meaning as defined above) or R³¹ is ethynyl or -C≡C-R^{26a} (wherein R^{26a} has the same meaning as defined above) may be produced from the compounds in which R⁹ or R³¹ is formyl or substituted or unsubstituted lower alkanoyl in a known method [Jikken Kagaku Kohza (Manual for Experimental Chemistry), 4th edition, Edited by Chemical Society of Japan, vol. 19, pp. 298-312 (1991)] or a similar method thereto.

The compounds in which R⁹ is -CH₂NR²³R²⁴ (wherein R²³ and R²⁴ each have the same meaning as defined above) or R³¹ is -CH₂NR^{23a}R^{24a} (wherein R^{23a} and R^{24a} each have the same meaning as defined above) may be produced from the compounds in which R⁹ or R³¹ is formyl in a known method [Jikken Kagaku Kohza (Manual for Experimental Chemistry), 4th edition, Edited by Chemical Society of Japan, vol. 20, pp. 300-302 (1991); idem, vol. 22, pp. 138-148 or pp. 166-167 (1991); idem, vol. 24, pp. 394-397 (1991)] or a similar method thereto.

The compounds in which R⁹ is -CONR²⁹R³⁰ (wherein R²⁹ and R³⁰ each have the same meaning as defined above) or R³¹ is -CONR^{27d}R^{28d} (wherein R^{27d} and R^{28d} each have the same meaning as defined above) may be produced from the compounds in which R⁹ or R³¹ is carboxy in a known method [Jikken Kagaku Kohza (Manual for Experimental Chemistry), 4th edition, Edited by Chemical Society of Japan, vol. 22, pp. 138-148 (1991)] or a similar method thereto.

The compounds in which R⁹ is -CH=CHCONR^{27a}R^{28a} (wherein R^{27a} and R^{28a} each have the same meaning as defined above) or R³¹ is -CH=CHCONR^{27c}R^{28c} (wherein R^{27c} and R^{28c} each have the meaning as defined above) may be produced by converting the compounds in which R⁹ or R³¹ is formyl into the compounds in which R⁹ or R³¹ is -CH=CHCOOR⁴³ (wherein R⁴³ represents the lower alkyl as defined above) in a known method [Jikken Kagaku Kohza (Manual for Experimental Chemistry), 4th edition, Edited by Chemical Society of Japan, vol. 19, pp. 57-74 (1991)] or a similar method thereto, and then by subjecting the resulting compound to a known method [Jikken Kagaku Kohza (Manual for Experimental Chemistry), 4th edition, Edited by Chemical Society of Japan, vol. 22, pp. 148-150 (1991)] or a similar method thereto.

The compounds in which R⁹ is -CH₂CONR²⁷R²⁸ (wherein R²⁷ and R²⁸ each have the same meaning as defined above) or R³¹ is -CH₂CONR^{27b}R^{28b} (wherein R^{27b} and R^{28b} each have the same meaning as defined above) may be produced from the compounds in which R⁹ or R³¹ is -CH₂COOR^{16b} (wherein R^{16b} has the same meaning as R^{16a} defined above) prepared in the step 2, in a known method [Jikken Kagaku Kohza (Manual for Experimental Chemistry), 4th edition, Edited by Chemical Society of Japan, vol. 22, pp. 148-150 (1991)] or a similar method thereto.

The compounds in which R⁹ or R³¹ is -(CH₂)_{qb}CONR²⁷R²⁸ (wherein R²⁷ and R²⁸ each have the same meaning as defined above; and qb indicates 2 or 3) may be produced by converting the compounds in which R⁹ or R³¹ is formyl into the compounds in which R⁹ or R³¹ is -(CH₂)_{qc}CHO (wherein qc indicates 1 or 2) in a known method [Jikken Kagaku Kohza (Manual for Experimental Chemistry), 4th edition, Edited by Chemical Society of Japan, vol. 21, pp. 124-133 (1991)] or a similar method thereto, then converting the latter into the compounds in which R⁹ or R³¹ is -(CH₂)_{qd}COOH (wherein qd indicates 1 or 2) in a known method [Jikken Kagaku Kohza (Manual for Experimental Chemistry), 4th edition, Edited by Chemical Society of Japan, vol. 22, pp. 21-25 (1991)] or a similar method thereto, and then by subjecting the resulting compound to a known method [Jikken Kagaku Kohza (Manual for Experimental Chemistry), 4th edition, Edited by Chemical Society of Japan, vol. 22, pp. 138-148 (1991)] or a similar method thereto.

In the above-described respective production processes, the intermediates and the desired compounds may be isolated and purified by means of separation and purification conventionally utilized in synthetic organic chemistry, for example, filtration, extraction, washing, drying, concentration, recrystallization, a variety of chromatography, or the like. The intermediates may also be subjected to the subsequent reaction without purification.

Compounds (I) may exist in the form of stereoisomers such as geometrical isomers or optical isomers, and the present invention encompasses all isomers including these isomers and mixtures of any possible isomers at any rate.

When it is desired to obtain Compound (I) as a salt, and it is obtained as a salt, Compound (I) may be purified as such. When Compound (I) is obtained in a free form, it may be dissolved or suspended in a suitable solvent, followed by addition of an acid to form the salt.

In addition, Compounds (I) and their pharmaceutically acceptable salts may exist in the form of adducts with water or a variety of solvents, which are also included in the present invention.

Table 1 shows the specific examples of Compounds (I) prepared in the present invention.

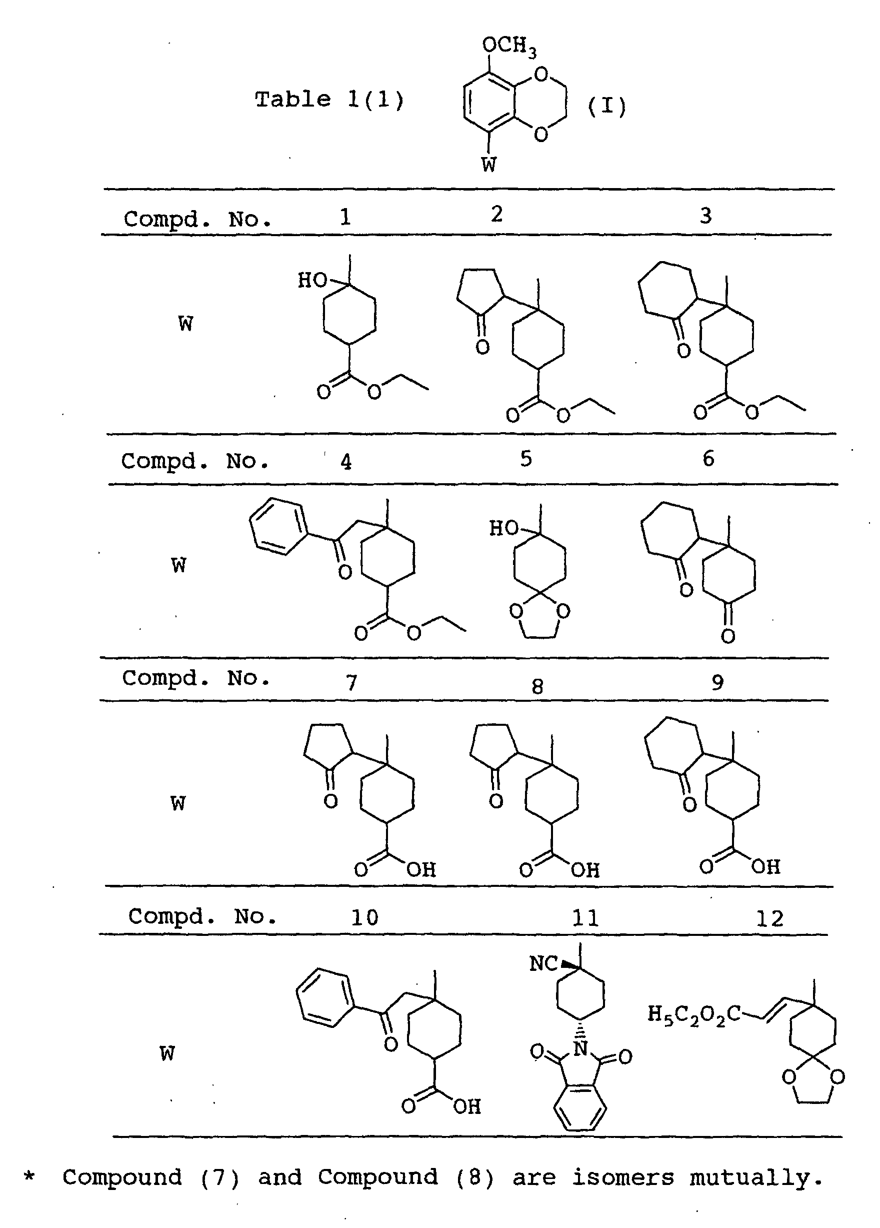

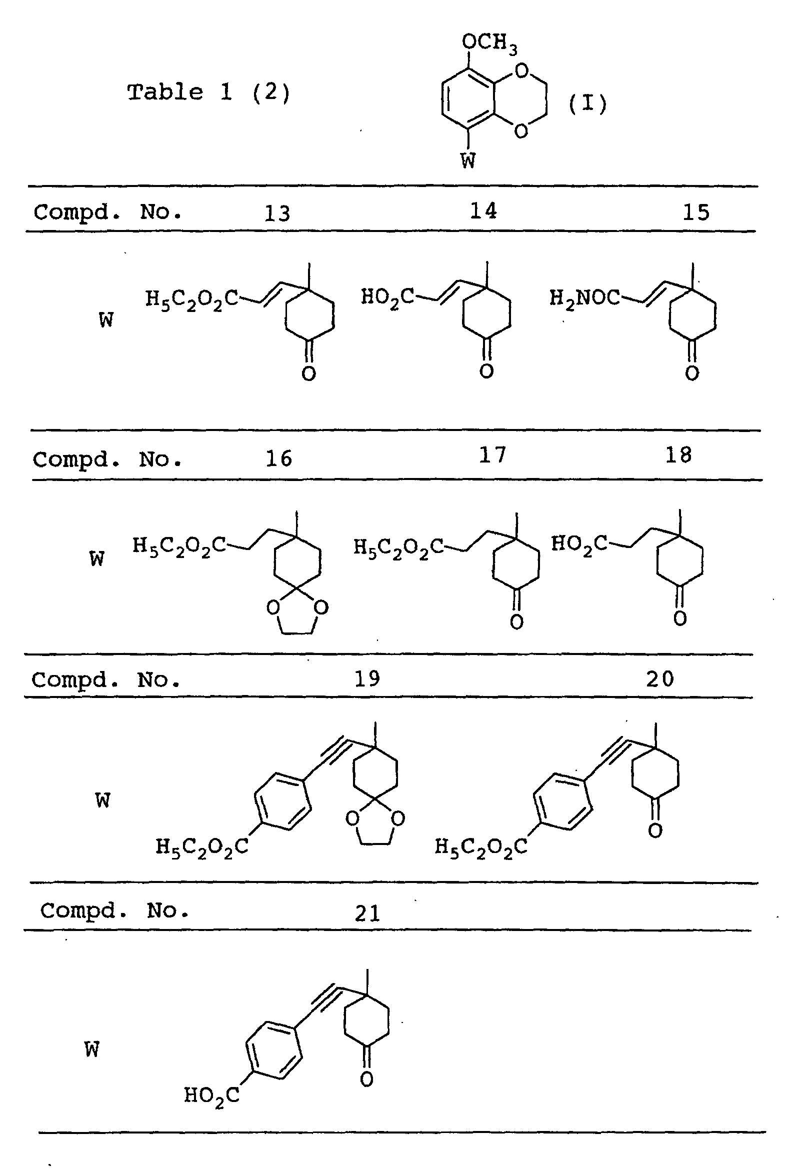

The following test examples will specifically explain the pharmacological effects of the typical Compounds (I).

### Test Example 1: Inhibition test on recombinant human PDE IV enzyme

Human phosphodiesterase cDNA (HSPDE4A4B) was isolated from HL-60 cells. The expected amino acid sequence is the same as that (HSPDE4A5) reported by G. Bolger et al. [Mol. Cell Biol. 13, p. 6558 (1993)]. This recombinant protein was expressed in Sf9 insect cells. The PDE activity was determined by the following two step process according to the method by R.L. Kincaid and V.C. Manganiello [Method. Enzymol., 159, pp. 457-470 (1988)]. Using [³H]cAMP (final concentration: 1µmol/L) as a substrate, the reaction was conducted in a standard mixture containing N,N-bis(2-hydroxyethyl)-2-aminoethanesulfonic acid (50 mmol/L, pH 7.2), MgCl₂ (1 mmol/L) and soybean tryps in inhibitor (0.1 mg/mL). The reaction was started by addition of the enzyme, and the incubation was conducted at 30°C for 10 to 30 minutes. The reaction was terminated with hydrochloric acid, and the formed 5'-AMP was completely decomposed with 5'-nucleosidase. After chromatography with DEAE-Sephadex A-25, the eluted [³H]-adenosine was counted by a scintillation counter. The test compounds were added after dissolved in DMSO (concentration: 1.7%).

As a result, it was found that Compound 8 and Compound 11 showed 30% or more enzyme inhibitory activity at a drug concentration of 10 µmol/L, and Compound 13 showed 99% or more enzyme inhibitory activity at a concentration of 1 µmol/L.

It is usually desirable to administer Compounds (I) or pharmaceutically acceptable salts thereof in the form of various pharmaceutical preparations, though they can be administered as they are. Such pharmaceutical preparations can be used for animals and humans.

The pharmaceutical preparations of the present invention may contain Compound (I) or a pharmaceutically acceptable salt thereof as an active component, alone or in a mixture with other therapeutically effective component. Further, the pharmaceutical preparations may be prepared by any means which are well known in the technical filed of pharmaceutics after mixing the active component with one or more pharmaceutically acceptable carriers.

Examples of the effective component to be mixed are serotonin (5HT)₃ receptor antagonists, serotonin (5HT)₄ receptor agonists, serotonin (5HT)_{1A} receptor agonists, dopamine (D)₂ receptor antagonists, histamine (H)₁ receptor antagonists, muscarine receptor antagonists, neurokinin (NK)₁ receptor antagonists and endothelin (ET)_{A} receptor antagonists.

As for the administration route, it is desirable to use the most effective route for therapy, including oral administration and parenteral administration, for example, intrabuccal, intratracheal, intrarectal, subcutaneous, intramuscular and intravenous administrations.

The administration form includes sprays, capsules, tablets, granules, syrups, emulsions, suppositories, injections, ointments, tapes, and the like.

Liquid preparations such as emulsions and syrups, which are suitable for oral administration can be prepared using water; sugars such as sucrose, sorbitol or fructose; glycols such as polyethylene glycol or propylene glycol; oils such as sesame oil, olive oil or soybean oil; preservatives such as p-hydroxybenzoate; flavors such as strawberry flavor or peppermint flavor; or the like. Capsules, tablets, powder and granules can be prepared using excipients such as lactose, glucose, sucrose or mannitol; disintegrators such as starch or sodium alginate; lubricants such as magnesium stearate or talc; binders such as polyvinyl alcohol, hydroxypropyl cellulose or gelatine; surfactants such as fatty acid esters; plasticizers such as glycerin; or the like.

Preparations suitable for parenteral administration comprise a sterilized aqueous preparation containing the active compound, which is preferably isotonic to the blood of a recipient. For example, a solution for injection may be prepared using a carrier such as a salt solution, a glucose solution, or a mixture of a salt solution and glucose solution. Preparations for intrarectal administration may be prepared using a carrier such as cacao fat, hydrogenated fat or hydrogenated carboxylic acid, and provided as suppositories. Sprays may be prepared using the active compound itself or together with carriers which can disperse the active compound as fine particles to facilitate absorption without stimulating oral or respiratory mucosa. Examples of such carriers include lactose, glycerin, and the like. Depending on the properties of the active compound and carriers used, preparations such as aerosol and dry powder can be prepared.

These parenteral preparations may also contain one or more auxiliary components selected from diluents, flavors, preservations, excipients, disintegrators, lubricants, binders, surfactants, plasticizers, and the like, all of which are mentioned in the above oral preparations.

The effective dose and administration schedule of Compounds (I) or their pharmaceutically acceptable salts may vary depending on the administration form, the age and body weight of a patient, and the type or degree of the disease to be treated, but usually in the case of oral administration, Compounds (I) or their pharmaceutically acceptable salts are administered in a dose of 0.01 mg - 1 g/adult/day, preferably 0.05-50 mg/adult/day, at one time or in several parts. In the case of parenteral administration such as intravenous administration, Compounds (I) or their pharmaceutically acceptable salts are administered in a dose of 0.001-100 mg/adult/day, preferably 0.01-10 mg/adult/day, at one time or in several parts . However, the doses vary depending on the various conditions described above.

The following examples will describe the embodiment of the present invention.

### Best Mode for Carrying Out the Invention

### Example 1

### Ethyl 4-hydroxy-4-(8-methoxy-1,4-benzodioxan-5-yl)cyclohexanecarboxylate (Compound 1)

5-Bromo-8-methoxy-1,4-benzodioxane (15 g, 61 mmol) was dissolved in THF (100 mL), to which was dropwise added a 1.6 mol/L n-butyllithium hexane solution (40 mL, 64 mmol) at -78°C. After 10 minutes, this solution was dropwise added to a solution of 4-ethoxycarbonylcyclohexanone (13 mL, 80 mmol) in THF (190 mL) at -78°C. After 10 minutes, the reaction mixture was added to 1 mol/L hydrochloric acid. The mixture was extracted with ethyl acetate, and the extract was washed with saturated brine and dried over sodium sulfate. The solvent was distilled off, and the residue was purified by silica gel column chromatography (eluted with hexane/ethyl acetate= 2/1) to yield Compound 1 (8.9 g, 42%) as a light yellow oil (a mixture of isomers). A portion of the product was subjected to preparative thin layer chromatography (developed with diethyl ether/ethyl acetate= 10/1) to separate both isomers.

### The isomer with the high Rf value

¹H-NMR (CDCl₃, δ, ppm) 1.27 (t, J = 7 Hz, 3H), 1.70-2.20 (m, 8H), 2.20-2.40 (m, 1H), 3.37 (s, 1H), 3.86 (s, 3H), 4.15 (q, J = 7 Hz, 2H), 4.35 (s, 4H), 6.47 (d, J = 9 Hz, 1H), 6.81 (d, J = 9 Hz, 1H).

### The isomer with the low Rf value

¹H-NMR (CDCl₃, δ, ppm) 1.25 (t, J = 7 Hz, 3H), 1.72-2.23 (m, 8H), 2.52-2.70 (m, 1H), 3.54 (s, 1H), 3.86 (s, 3H), 4.14 (q, J = 7 Hz, 2H), 4.34 (s, 4H), 6.46 (d, J = 9 Hz, 1H), 6.82 (d, J = 9 Hz, 1H).

### Example 2

### Ethyl 4-(cyclopentanon-2-yl)-4-(8-methoxy-1,4-benzodioxan-5-yl)cyclohexanecarboxylate (Compound 2)

Compound 1 (1.5 g, 4.5 mmol) prepared in Example 1 was dissolved in dichloromethane (15 mL), to which was dropwise added 1-trimethylsiloxycyclopentene (1.2 mL, 6.6 mmol) at -78°C, and after 10 minutes, a boron trifluoride/diethyl ether complex (0.81 mL, 6.6 mmol) was dropwise added thereto. After 5 minutes, the reaction mixture was added to an aqueous sodium bicarbonate solution and the mixture was extracted with chloroform. The extract was washed with saturated brine and dried over magnesium sulfate, and the solvent was evaporated. The residue was purified by silica gel column chromatography (eluted with hexane/ethyl acetate= 4/1) to yield the isomer with the high Rf value (Compound 2a)(0.45 g, 25%) and the isomer with the low Rf value (Compound 2b)(0.19 g, 11%), both as a colorless oil.
Compound 2a
   ¹H-NMR (CDCl₃, δ, ppm) 1.19 (t, J = 7 Hz, 3H), 1.20-2.42 (m, 14H), 2.50-2.88 (m, 2H), 3.87 (s, 3H), 4.05 (q, J= 7 Hz, 2H), 4.10-4.25 (m, 2H), 4.25-4.38 (m, 2H), 6.45 (d, J = 9 Hz, 1H), 6.75 (d, J = 9 Hz, 1H).
Compound 2b
   ¹H-NMR (CDCl₃, δ, ppm) 1.28 (t, J = 7 Hz, 3H), 1.41-2.48 (m, 15H), 2.52-2.70 (m, 1H), 3.87 (s, 3H), 4.10-4.25 (m, 4H), 4.25-4.35 (m, 2H), 6.46 (d, J = 9 Hz, 1H), 6.78 (d, J = 9 Hz, 1H).

### Example 3

### Ethyl 4-(cyclohexanon-2-yl)-4-(8-methoxy-1,4-benzodioxan-5-yl)cyclohexanecarboxylate (Compound 3)

In a manner similar to that in Example 2, using 1-trimethylsiloxycyclohexene instead of 1-trimethylsiloxycyclopentene, Compound 1 (1.3 g, 3.9 mmol) prepared in Example 1 was converted into Compound 3 (1.1 g, 69%) in a colorless oil (a 2:1 mixture of isomers).
¹H-NMR (CDCl₃, δ, ppm) 1.19 and 1.30 (each t, J = 7 Hz, total 3H), 1.35-2.52 and 2.69-2.82 (each m, total 17H), 2.85-3.04(m, 1H), 3.86 and 3.87 (each s, total 3H), 4.04 (q, J = 7 Hz, 1.4H), 4.10-4.39 (m, 4.6H), 6.46 and 6.48 (each d, J = 9 Hz, total 1H), 6.84 and 6.85 (each d, J = 9 Hz, total 1H).

### Example 4

### Ethyl 4-(8-methoxy-1,4-benzodioxan-5-yl)-4-(1-phenylethanon-2-yl)cyclohexanecarboxylate (Compound 4)

In a manner similar to that in Example 2, using 1-phenyl-1-trimethylsiloxyethylene instead of 1-trimethylsiloxycyclopentene, Compound 1 (1.5 g, 4.5 mmol) prepared in Example 1 was converted into Compound 4 (1.1 g, 55%) in a colorless oil (a 2:1 mixture of isomers).
¹H-NMR (CDCl₃, δ, ppm) 1.20 and 1.27 (each t, J = 7 Hz, total 3H), 1.40-2.00(m,6H), 2.22-2.42 (m,1H), 2.48-2.64 and 2.72-2.95 (each m, total 2H), 3.28 and 3.58 (each s, total 2H), 3.80 (s, 3H), 3.95-4.29 (m, 6H), 6.37 and 6.40 (each d, J = 9 Hz, total 1H), 6.72 and 6.76 (each d, J = 9 Hz, total 1H), 7.22-7.38 (m, 2H), 7.39-7.52 (m, 1H), 7.65-7.78 (m, 2H).

### Example 5

### 4-Hydroxy-4-(8-methoxy-1,4-benzodioxan-5-yl)cyclohexanone ethylene ketal (Compound 5)

5-Bromo-8-methoxy-1,4-benzodioxane (10 g, 41 mmol) was dissolved in THF (65 mL), to which was dropwise added a 1.6 mol/L n-butyllithium hexane solution (28 mL, 45 mmol) at -78°C. After 15 minutes, a solution of 1,4-cyclohexadione monoethylene ketal (9.6 g, 61 mmol) in THF (50 mL) was dropwise added thereto, and after stirring for 1 hour, the mixture was stirred at room temperature for 20 minutes. Water was added thereto, and the mixture was extracted with ethyl acetate. The extract was washed with saturated brine and dried over sodium sulfate, and the solvent was evaporated. The residue was purified by silica gel column chromatography (eluted with hexane/ethyl acetate= 1/1) to yield Compound 5 (9.0 g, 68%) as a white solid.
Melting Point: 94-96 °C
¹H-NMR (CDCl₃, δ, ppm) 1.58-1.72 (m, 2H), 1.88-2.28 (m, 6H), 3.57 (s, 1H), 3.86 (s, 3H), 3.90-4.07 (m, 4H), 4.35 (s, 4H), 6.46 (d, J = 9 Hz, 1H), 6.82 (d, J = 9 Hz, 1H).
MASS (m/z) 322 (M⁺).

### Example 6

### 4-(Cyclohexanon-2-yl)-4-(8-methoxy-1,4-benzodioxan-5-yl)-cyclohexanone (Compound 6)

Compound 5 (0.6 g, 1.9 mmol) prepared in Example 5 was dissolved in dichloromethane (6.0 mL), to which was dropwise added 1-trimethylsiloxycyclohexene (0.54 mL, 2.8 mmol) at -78°C, and after 10 minutes, a boron trifluoride/diethyl ether complex (0.34 mL, 2.8 mmol) was dropwise added thereto. After 5 minutes, the reaction mixture was added to an aqueous sodium bicarbonate solution and the mixture was extracted with chloroform. The extract was washed with saturated brine and dried over magnesium sulfate, and the solvent was evaporated. The residue was dissolved in acetone (6.0 mL), to which was added 2.0 mol/L hydrochloric acid (2.4 mL), and the mixture was stirred at room temperature for 17 hours. A saturated aqueous sodium bicarbonate solution was added thereto and the mixture was extracted with ethyl acetate. The extract was washed with saturated brine and dried over magnesium sulfate, and the solvent was evaporated. The residue was purified by silica gel column chromatography (eluted with hexane/ethyl acetate= 2/1) and recrystallized from acetone to yield Compound 6 (0.22 g, 33%) as a white solid.
Melting Point: 268-269 °C
¹H-NMR (CDCl₃, δ, ppm) 0.60-0.80 (m, 1H), 1.20-1.85 (m, 9H), 2.01 (dd, J = 1, 12 Hz, 1H), 2.40-2.49 (m, 3H), 2.52 (d, J = 5 Hz, 1H), 2.75-2.90 (dt, J = 3, 12 Hz, 1H), 2.90-3.10 (m, 1H), 3.86 (s, 3H), 4.15-4.45 (m, 4H), 6.43 (d, J = 9 Hz, 1H), 6.54 (d, J = 9 Hz, 1H).

| Elemental analysis:C₂₁H₂₆O₅ | |
|---|---|
| Found (%) | C : 70.03, H : 7.37 |
| Calcd. (%) | C : 70.37, H : 7.31 |

### Example 7

### 4-(Cyclopentanon-2-yl)-4-(8-methoxy-1,4-benzodioxan-5-yl)-cyclohexanecarboxylic acid (Compound 7)

Compound 2a (0.45 g, 1.1 mmol) prepared in Example 2 was dissolved in ethanol (4.5 mL), to which was added a 1 mol/L aqueous sodium hydroxide solution (7.2 mL), and the mixture was stirred at room temperature for 24 hours. The mixture was then acidified with 6 mol/L hydrochloric acid under ice-cooling, and extracted with ethyl acetate. The extract was washed with saturated brine and dried over magnesium sulfate, and the solvent was evaporated. The residue was recrystallized from ethanol to yield Compound 7 (0.27 g, 67%) as a white solid.
Melting Point: 200-201 °C
¹H-NMR (DMSO-d₆, δ, ppm) 1.00-1.79 (m, 12H), 1.82-2.26 (m, 2H), 2.40-2.80 (m, 2H), 3.73 (s, 3H), 4.00-4.30 (m, 4H), 6.52 (d, J = 9 Hz, 1H), 6.69 (d, J = 9 Hz, 1H).

| Elemental analysis:C₂₁H₂₆O₆ | |
|---|---|
| Found (%) | C : 67.62, H : 7.02 |
| Calcd. (%) | C : 67.36, H : 7.00 |

### Example 8

### 4-(Cyclopentanon-2-yl)-4-(8-methoxy-1,4-benzodioxan-5-yl)-cyclohexanecarboxylic acid (Compound 8)

In a manner similar to that in Example 7, Compound 2b (0.19 g, 0.47 mmol) prepared in Example 2 was converted into Compound 8 (0.10 g, 57%) in a white solid.
Melting Point: 241-243 °C
¹H-NMR (DMSO-d₆, δ , ppm) 1.20-2.65 (m, 16H), 3.72 (s, 3H), 4.00-4.27 (m, 4H), 6.50 (d, J = 9 Hz, 1H), 6.69 (d, J = 9 Hz, 1H).

| Elemental analysis:C₂₁H₂₆O₆ | |
|---|---|
| Found (%) | C : 67.56, H : 7.31 |
| Calcd. (%) | C : 67.36, H : 7.00 |

### Example 9

### 4-(Cyclohexanon-2-yl)-4-(8-methoxy-1,4-benzodioxan-5-yl)-cyclohexanecarboxylic acid (Compound 9)

In a manner similar to that in Example 7, Compound 9 (0.93 g, 100%) was obtained as an isomeric mixture using Compound 3 (1.0 g, 2.4 mmol) prepared in Example 3. The obtained mixture was subjected to recrystallization from ethanol to separate the respective isomers (Compound 9a and Compound 9b).
Compound 9a
Melting Point: 243-244 °C
¹H-NMR (DMSO-d₆, δ, ppm) 1.00-2.43 (m, 16H), 2.57-2.78 (m, 1H), 2.83-3.05 (m, 1H), 3.73 (s, 3H), 4.08-4.30 (m, 4H), 6.53 (d, J = 9 Hz, 1H), 6.71 (d, J = 9 Hz, 1H).

| Elemental analysis:C₂₂H₂₈O₆ | |
|---|---|
| Found (%) | C : 68.15, H : 7.26 |
| Calcd. (%) | C : 68.02, H : 7.27 |

Compound 9b
Melting Point: 222-223 °C
¹H-NMR (DMSO-d₆, δ, ppm) 1.20-2.44 (m, 17H), 2.72-2.90 (m, 1H), 3.73 (s, 3H), 4.05-4.27 (m, 4H), 6.52 (d, J = 9 Hz, 1H), 6.72 (d, J = 9 Hz, 1H).

### Example 10

### 4-(8-Methoxy-1,4-benzodioxan-5-yl)-4-(1-phenylethanon-2-yl)cyclohexanecarboxylic acid (Compound 10)

In a manner similar to that in Example 7, Compound 4 (1.1 g, 2.4 mmol) prepared in Example 4 was converted into Compound 10 (0.16 g, 16%) in a white solid.
Melting Point: 201-202 °C
¹H-NMR (CDCl₃, δ, ppm) 1.65-2.06 (m, 6H), 2.34-2.49 (m, 1H), 2.50-2.67 (m, 2H), 3.57 (s, 2H), 3.80 (s, 3H), 4.00-4.20 (m, 4H), 6.40 (d, J = 9 Hz, 1H), 6.75 (d, J = 9 Hz, 1H), 7.32 (dd, J = 8, 9 Hz, 2H), 7.44 (dd, J = 1, 8 Hz, 1H), 7.70 (dd, J = 1, 9 Hz, 2H).

| Elemental analysis:C₂₄H₂₆O₆ | |
|---|---|
| Found (%) | C : 70.35, H : 6.42 |
| Calcd. (%) | C : 70.23, H : 6.38 |

### Example 11

### trans-4-Cyano-4-(8-methoxy-1,4-benzodioxan-5-yl)-1-phthalimidocyclohexane (Compound 11)

Compound A (0.6 g, 2.1 mmol) prepared in Reference Example was dissolved in THF (18 mL), to which were added triphenylphosphine (0.82 g, 3.1 mmol) and phthalimide (0.46 g, 3.1 mmol). Then, a 40% diethyl azodicarboxylate/toluene solution (1.4 mL, 3.1 mmol) was dropwise added thereto under ice cooling, and the mixture was stirred at room temperature for 3 hours. The solvent was distilled off, and the residue was purified by silica gel column chromatography (eluted with hexane/acetone= 3/1) and triturated with acetone to yield Compound 11 (0.58 g, 67%) as a white solid.
¹H-NMR (CDCl₃, δ, ppm) 1.70-1.87 (m, 2H), 2.15 (dt, J = 4, 14 Hz, 2H), 2.30-2.52 (m, 2H), 2.88-3.05 (m, 2H), 3.92 (s, 3H), 4.22-4.42 (m, 1H), 4.37 (s, 4H), 6.59 (d, J = 9 Hz, 1H), 6.95 (d, J = 9 Hz, 1H), 7.60-7.88 (m, 4H).

| Elemental analysis:C₂₄H₂₂N₂O₅·0.4H₂O | |
|---|---|
| Found (%) | C : 67.69, H : 5.72, N : 6.30 |
| Calcd. (%) | C : 67.72, H : 5.40, N : 6.58 |

### Example 12

### Ethyl 3-[8-(8-methoxy-1,4-benzodioxan-5-yl)-1,4-dioxaspiro[4.5]dec-8-yl]acrylate (Compound 12)

In an argon atmosphere, potassium tert-butoxide (0.51 g, 4.6 mmol) was added to a solution of triethyl phosphonoacetate (0.91 mL, 4.6 mmol) in THF (18 mL) under ice-cooling, and the mixture was stirred at room temperature for 30 minutes. The reaction mixture was ice-cooled, to which was added a solution of Compound B (0.51 g, 1.5 mmol; prepared in Reference Example 2) in THF (5 mL), and the mixture was stirred at room temperature for 4 hours. The reaction mixture was poured into water and the mixture was extracted with ethyl acetate. The organic layer was washed with water and then with saturated brine and dried over sodium sulfate, and the solvent was evaporated under reduced pressure. The residue was purified by silica gel column chromatography (eluted with hexane/ethyl acetate= 2/1) to yield Compound 12 (0.57 g, 93%) as a colorless oil.
¹H-NMR (CDCl₃, δ, ppm) 1.26 (t, J = 7 Hz, 3H), 1.65-1.80 (m, 4H), 2.04-2.28 (m, 4H), 3.86 (s, 3H), 3.90-3.99 (m, 4H), 4.15 (q, J = 7 Hz, 2H), 4.11-4.31 (m, 4H), 5.61 (d, J = 16 Hz, 1H), 6.44 (d, J = 9 Hz, 1H), 6.78 (d, J = 9 Hz, 1H), 7.27 (d, J = 16 Hz, 1H).

### Example 13

### Ethyl 3-[1-(8-methoxy-1,4-benzodioxan-5-yl)-4-oxocyclohexyl]acrylate (Compound 13)

To a solution of Compound 12 (0.21 g, 0.5 mmol) prepared in Example 12 in acetone (3 mL) was added 2 mol/L hydrochloric acid (2.6 mL, 5.2 mmol), and the mixture was stirred at 60°C for 1.7 hours. The reaction mixture was poured into a saturated aqueous sodium bicarbonate solution and the mixture was extracted with ethyl acetate. The organic layer was washed with water and then with saturated brine and dried over sodium sulfate, and the solvent was evaporated under reduced pressure. The residue was recrystallized twice from ethanol to yield Compound 13 (0.08 g, 43%) as a colorless solid.
¹H-NMR (CDCl₃, δ, ppm) 1.28 (t, J = 7 Hz, 3H), 2.27-2.61 (m, 8H), 3.87 (s, 3H), 4.18 (q, J = 7 Hz, 2H), 4.21-4.34 (m, 4H), 5.71 (d, J = 16 Hz, 1H), 6.49 (d, J = 9 Hz, 1H), 6.80 (d, J = 9 Hz, 1H), 7.29 (d, J = 16 Hz, 1H).

### Example 14

### 3-[1-(8-Methoxy-1,4-benzodioxan-5-yl)-4-oxocyclohexyl]-acrylic acid (Compound 14)

To a solution of Compound 13 (1.29 g, 3.58 mmol; prepared in Example 13) in ethanol (13 mL) was added a 2 mol/L aqueous sodium hydroxide solution (9.0 mL, 18 mmol), and the mixture was stirred at room temperature for 4 hours. Ethanol was distilled off under reduced pressure, and the resulting mixture was acidified with 6 mol/L hydrochloric acid and extracted with ethyl acetate. The organic layer was washed twice with water and then with saturated brine and dried over sodium sulfate, and the solvent was evaporated under reduced pressure to yield Compound 14 (1.23 g, quantitative yield) as a colorless amorphous solid.
¹H-NMR (CDCl₃, δ, ppm) 2.28-2.59 (m, 8H), 3.88 (s, 3H), 4.20-4.33 (m, 4H), 5.71 (d, J = 16 Hz, 1H), 6.50 (d, J = 9 Hz, 1H), 6.80 (d, J = 9 Hz, 1H), 7.38 (d, J = 16 Hz, 1H) (1H for carboxy was not detected).
MASS (m/z) 331 (M⁻-1).

### Example 15

### 3-[1-(8-Methoxy-1,4-benzodioxan-5-yl)-4-oxocyclohexyl]-acrylamide (Compound 15)

To a solution of Compound 14 (0.20 g, 0.6 mmol; prepared in Example 14) in N,N-dimethylformamide (4 mL) were added 1-hydroxybenzotriazole (HOBt) ammonium salt (0.14 g, 0.9 mmol) and 1-(3-dimethylaminopropyl)-3-ethylcarbodiimide (WSC) hydrochloride (0.23 g, 1.2 mmol), and the mixture was stirred at room temperature overnight. After ice-cooling, water was added thereto, and the mixture was extracted with ethyl acetate. The organic layer was washed with a saturated aqueous sodium bicarbonate solution, water, and saturated brine and dried over sodium sulfate, and the solvent was evaporated under reduced pressure. The residue was purified by preparative thin layer chromatography (twice developed with chloroform/methanol= 20/1) to yield Compound 15 (96 mg, 48%) as a colorless solid.
¹H-NMR (CDCl₃, δ, ppm) 2.24-2.58 (m, 8H), 3.87 (s, 3H), 4.20-4.33 (m, 4H), 5.37 (brs, 2H), 5.66 (d, J = 15 Hz, 1H), 6.49 (d, J = 9 Hz, 1H), 6.81 (d, J = 9 Hz, 1H), 7.25(d, J = 16 Hz, 1H). MASS (m/z) 332 (M⁺+1).

### Example 16

### Ethyl 3-[8-(8-methoxy-1,4-benzodioxan-5-yl)-1,4-dioxaspiro[4.5]dec-8-yl]propionate (Compound 16)

To a solution of Compound 12 (0.2 g, 0.5 mmol; prepared in Example 12) in ethanol (2 mL) was added 10% palladium-carbon (50% wet with water)(53 mg), and the mixture was subjected to hydrogenation at ordinary temperature under atmospheric pressure overnight. The catalyst was removed, and the solvent was evaporated from the filtrate under reduced pressure. The residue was purified by silica gel column chromatography (eluted with hexane/ethyl acetate= 1/1) to yield Compound 16 (0.57 g, 93%) as a colorless oil.
¹H-NMR (CDCl₃, δ, ppm) 1.18 (t, J = 7 Hz, 3H), 1.5-2.2 (m, 10H), 2.3-2.4 (m, 2H), 3.85 (s, 3H), 3.86-3.97 (m, 4H), 4.01 (q, J = 7 Hz, 2H), 4.21-4.32 (m, 4 H), 6.42 (d, J = 9 Hz, 1H), 6.67 (d, J = 9 Hz, 1H).

### Example 17

### Ethyl 3-[1-(8-methoxy-1,4-benzodioxan-5-yl)-4-oxocyclohexyl]propionate (Compound 17) and 3-[1-(8-methoxy-1,4-benzodioxan-5-yl)-4-oxocyclohexyl]propionic acid (Compound 18)

To a solution of Compound 16 (0.20 g, 0.5 mmol; prepared in Example 16) in acetone (4 mL) was added 2 mol/L hydrochloric acid (2.4 mL, 4.8 mmol), and the mixture was stirred at 50°C for 2 hours. The reaction mixture was added into a saturated aqueous sodium bicarbonate solution and the mixture was extracted with ethyl acetate. The organic layer was washed with water and then with saturated brine and dried over sodium sulfate, and the solvent was evaporated under reduced pressure. The residue was purified by silica gel column chromatography (eluted with hexane/ethyl acetate= 1/1) to yield Compound 17 (64 g, 36%) as a white solid. The aqueous layer obtained by the above extraction with ethyl acetate was acidified with 6 mol/L hydrochloric acid, and then extracted with ethyl acetate. The organic layer was washed with water and then with saturated brine and dried over sodium sulfate, and the solvent was evaporated under reduced pressure to yield Compound 18 (90 mg, 55%).
Compound 17
   ¹H-NMR (CDCl₃, δ, ppm) 1.20 (t, J= 7 Hz, 3H), 1.84-1.95 (m, 2H), 1.99-2.05 (m, 2H), 2.11-2.18 (m, 2H), 2.26-2.41 (m, 4H), 2.65-2.70 (m, 2H), 3.87 (s, 3H), 4.03 (q, J = 7 Hz, 2H), 4.26-4.35 (m, 4H), 6.48 (d, J = 9 Hz, 1H), 6.72 (d, J = 9 Hz, 1H).
Compound 18
   ¹H-NMR (CDCl₃, δ, ppm) 1.83-1.94 (m, 2H), 2.03-2.19 (m, 4H), 2.26-2.41 (m, 4H), 2.66-2.71 (m, 2H), 3.87 (s, 3H), 4.24-4.34 (m, 4H), 6.48 (d, J = 9 Hz, 1H), 6.72 (d, J = 9 Hz, 1H) (1H of carbonate is not found).
   MASS (m/z) 333 (M⁻-1).

### Example 18

### Ethyl 4-[8-(8-methoxy-1,4-benzodioxan-5-yl)-1,4-dioxaspiro[4.5]dec-8-ylethynyl]benzoate (Compound 19)

In an argon atmosphere, bis(triphenylphosphine)palladium (II) chloride (64 mg, 0.09 mmol) and cuprous iodide (9 mg, 0.05 mmol) were added to a mixture of Compound C (0.3 g, 0.9 mmol) prepared in Reference Example 3, ethyl 4-iodobenzoate (0.3 g, 1.1 mmol) and diethylamine (3 mL), and the mixture was stirred at room temperature for 2.5 hours and then at 55°C for 2.2 hours. Ethyl acetate was added to the reaction mixture and the mixture was filtered through celite. The solvent was evaporated from the filtrate under reduced pressure, and the residue was purified by silica gel column chromatography (eluted with hexane/ethyl acetate= 2/1) to yield Compound 19 (0.28 g, 80%) as a brown oil. ¹H-NMR (CDCl₃, δ, ppm) 1.46 (t, J = 7 Hz, 3H), 1.77-1.81 (m, 2H), 2.14-2.41 (m, 6H), 3.87 (s, 3H), 3.99 (s, 4H), 4.31-4.41 (m, 6H), 6.48 (d, J = 9 Hz, 1H), 7.08 (d, J = 9 Hz, 1H), 7.44-7.50 (m, 2H), 7.93-7.98 (m, 2H).

### Example 19

### Ethyl 4-[1-(8-methoxy-1,4-benzodioxan-5-yl)-4-oxocyclohexylethynyl]benzoate (Compound 20)

To a solution of Compound 19 (0.28 g, 0.72 mmol; prepared in Example 18) in acetone (4.2 mL) was added 2 mol/L hydrochloric acid (3.6 mL, 7.2 mmol), and the mixture was stirred at 60°C for 3.7 hours. The reaction mixture was added into a saturated aqueous sodium bicarbonate solution and the mixture was extracted with ethyl acetate. The resulting organic layer was washed with a saturated aqueous sodium bicarbonate solution and then with saturated brine and dried over sodium sulfate, and the solvent was evaporated under reduced pressure. The residue was purified by silica gel column chromatography (eluted with hexane/ethyl acetate= 2/1) to yield Compound 20 (0.17 g, 55%) as a brown oil.
¹H-NMR (CDCl₃, δ, ppm) 1.40 (t, J= 7 Hz, 3H), 2.28-2.47 (m, 4H), 2.58-2.70 (m, 2H), 2.95-3.08 (m, 2H), 3.88 (s, 3H), 4.30-4.43 (m, 6H), 6.52 (d, J = 9 Hz, 1H), 7.22 (d, J = 9 Hz, 1H), 7.45-7.50 (m, 2H), 7.52-7.53 (m, 2H).

### Example 20

### 4-[1-(8-Methoxy-1,4-benzodioxan-5-yl)-4-oxocyclohexylethynyl]benzoic acid (Compound 21)

To a suspension of Compound 20 (0.15 g, 0.35 mmol; prepared in Example 19) in ethanol (3 mL) was added a 2 mol/L aqueous sodium hydroxide solution (1.7 mL, 3.5 mmol), and the mixture was stirred at room temperature for 6 hours. Insoluble material was removed by filtration, the filtrate was acidified with 2 mol/L hydrochloric acid, and the precipitated solid was collected by filtration and dried to yield Compound 21 (0.12 g, 84%) as a brown solid.
¹H-NMR (DMSO-d₆, δ, ppm) 2.27-2.5 (m, 6H), 2.84-2.94 (m, 2H), 3.74 (s, 3H), 4.2-4.3 (m, 4H), 6.58 (d, J = 9 Hz, 1H), 7.07 (d, J = 9 Hz, 1H), 7.61 (d, J = 8 Hz, 2H), 7.92 (d, J = 8 Hz, 2H) (1H for carboxy was not detected).
MASS (m/z) 405(M⁻-1).

### Reference Example 1

### cis-4-Cyano-4-(8-methoxy-1,4-benzodioxan-5-yl)cyclohexanol (Compound A)

### (Step A)

### 4-Cyano-4-(8-methoxy-1,4-benzodioxan-5-yl)cyclohexanone ethylene ketal (Compound Aa)

Compound 5 (0.49 g, 1.5 mmol) prepared in Example 5 was dissolved in methylene chloride (4.9 mL), to which was added trimethylsilyl cyanide (0.26 mL, 1.9 mmol) at -78°C, and then a boron trifluoride/diethyl ether complex (0.20 mL, 1.6 mmol) was dropwise added thereto. The mixture was stirred for 10 minutes and then at room temperature for 10 minutes. A saturated aqueous sodium bicarbonate solution was then added thereto, and the mixture was extracted with ethyl acetate. The extract was washed with saturated brine and dried over sodium sulfate, and the solvent was evaporated. The residue was purified by silica gel column chromatography (eluted with hexane/ethyl acetate= 2/1) to yield Compound Aa (0.30 g, 61%) as a colorless oil.
¹H-NMR (CDCl₃, δ, ppm) 1.79-1.95 (m, 2H), 2.06-2.20 (m, 4H), 2.30-2.46 (m, 2H), 3.87 (s, 3H), 3.90-4.07 (m, 4H), 4.36 (s, 4H), 6.48 (d, J = 9 Hz, 1H), 6.82 (d, J = 9 Hz, 1H).
MASS (m/z) 331 (M⁺).

### (Step B)

### 4-Cyano-4-(8-methoxy-1,4-benzodioxan-5-yl)cyclohexanone (Compound Ab)

Compound Aa (0.29 g, 0.87 mmol) prepared in Step A was dissolved in acetone (2.9 mL), to which was added 6 mol/L hydrochloric acid (1.2 mL, 7.2 mmol), and the mixture was refluxed under heating for 3 hours. After being allowed to stand for cooling, the mixture was poured into a saturated aqueous sodium bicarbonate solution and the mixture was extracted with ethyl acetate. The extract was washed with saturated brine and dried over sodium sulfate, and the solvent was evaporated to yield Compound Ab (0.23 g, 92%) as a white solid.
Melting Point: 157-161 °C
¹H-NMR (CDCl₃, δ, ppm) 2.21-2.41 (m, 2H), 2.45-2.72 (m, 4H), 2.81-3.00 (m, 2H), 3.89 (s, 3H), 4.37 (s, 4H), 6.51 (d, J = 9 Hz, 1H), 6.88 (d, J = 9 Hz, 1H).
MASS (m/z) 287 (M⁺).

### (Step C)

### Compound A

Compound Ab (0.42 g, 1.5 mmol) prepared in Step B was dissolved in methanol (8.4 mL), to which was added sodium borohydride (0.11 g, 3.0 mmol) under ice-cooling. After stirring at room temperature for 1 hour, again sodium borohydride (0.057 g, 1.5 mmol) was added thereto under ice-cooling, and the mixture was stirred at room temperture for 30 minutes. Then, 1 mol/L hydrochloric acid was added thereto under ice-cooling, and the mixture was extracted with ethyl acetate. The organic layer was washed with saturated brine and dried over sodium sulfate, and the solvent was evaporated under reduced pressure. The residue was purified by silica gel column chromatography (eluted with hexane/ethyl acetate= 1/2) and recrystallized from ethanol to yield Compound A (0.20 g, 48%) as white crystals.
Melting Point: 137-138 °C
¹H-NMR (CDCl₃, δ, ppm) 1.75-1.99 (m, 4H), 2.01-2.22 (m, 2H), 2.30-2.54 (m, 2H), 3.56-3.79 (m, 1H), 3.87 (s, 3H), 4.38 (s, 4H), 6.48 (d, J = 8 Hz, 1H), 6.83 (d, J = 8 Hz, 1H).
MASS (m/z) 289 (M⁺).

| Elemental analysis:C₁₆H₁₉NO₄·0.1H₂O | |
|---|---|
| Found (%) | C : 66.21, H : 6.94, N : 4.82 |
| Calcd. (%) | C : 66.01, H : 6.65, N : 4.81 |

### Reference Example 2

### 4-Formyl-4-(8-methoxy-1,4-benzodioxan-5-yl)cyclohexanone ethylene ketal (Compound B)

To a solution of Compound Aa (2.0 g, 6.0 mmol; prepared in Step A of Reference Example 1) in toluene (60 mL) was added a 1.0 mol/L diisobutylaluminum hydride/toluene solution (15.1 mL, 15.1 mmol) at room temperature, and the mixture was stirred overnight. After cooling the reaction mixture, a saturated aqueous sodium sulfate solution was added thereto, and the mixture was filtered. The precipitated solid was washed with ethyl acetate, and the solution obtained after washing was combined with the filtrate. The filtrate was dried over sodium sulfate, and the solvent was evaporated under reduced pressure. The residue was purified by silica gel column chromatography (eluted with hexane/ethyl acetate= 2/1) to yield Compound B (0.93 g, 46%) as a light yellowish white amorphous solid.
¹H-NMR (CDCl₃, δ, ppm) 1.69-2.11 (m, 4H), 2.01-2.11 (m, 2H), 2.27-2.35 (m, 2H), 3.87 (s, 3H), 3.9-4.0 (m, 4H), 4.20-4.31 (m, 4H), 6.50 (d, J = 9 Hz, 1H), 6.85 (d, J = 9 Hz, 1H), 9.56 (s, 1H).

### Reference Example 3

### 4-Ethenyl-4-(8-methoxy-1,4-benzodioxan-5-yl)cyclohexanone ethylene ketal (Compound C)

In an argon atmosphere, (bromomethyl)triphenylphosphonium bromide (1.0 g, 2.2 mmol) was added to a solution of potassium tert-butoxide (0.5 g, 4.5 mmol) in THF (10 mL) at -78°C and the mixture was stirred for 30 minutes. Then, a solution of Compound B (0.5 g, 1.5 mmol; prepared in Reference Example 2) in THF (8 mL) was dropwise added thereto, and the mixture was stirred at room temperature for 0.7 hours. Water was added to the reaction mixture and the resulting mixture was extracted with ethyl acetate. Then, water was added to the resulting organic layer, the mixture was then washed with saturated brine and dried over sodium sulfate, and the solvent was evaporated under reduced pressure. The residue was purified by silica gel column chromatography (eluted with hexane/ethyl acetate= 3/1) to yield Compound C (0.12 g, 24%) as a white solid.
¹H-NMR (CDCl₃, δ, ppm) 1.71-1.76 (m, 2H), 2.04-2.29 (m, 6H), 2.34 (s, 1H), 3.86 (s, 3H), 3.94-4.02 (m, 4H), 4.29-4.36 (m, 4H), 6.46 (d, J = 9 Hz, 1H), 7.08 (d, J = 9 Hz, 1H).
MASS (m/z) 331(M⁺+1).

### Industrial Applicability

The present invention provides oxygen-containing heterocyclic compounds which have a phosphodiesterase (PDE) IV inhibitory effect and are useful as a therapeutic agent for inflammatory allergic diseases such as bronchial asthma, allergic rhinitis, atopic dermatitis, nephritis, etc., autoimmune diseases such as chronic obstructive pulmonary disease, rheumatism, multiple sclerosis, Crohn's disease, psoriasis, systemic lupus erythematosus, etc., diseases in central nervous system such as depression, amnesia, dementia, etc., organ injury associated with ischemia reperfusion caused by heart failure, shock, cerebrovascular injury, etc., insulin-resistant diabetes, wound, AIDS, osteoporosis, urinary calculus, urinary incontinence, or the like, or as an agent for improving fatigue, lassitude, or the like.

## Claims

1. An Oxygen-containing heterocyclic compound represented by the following formula (I): 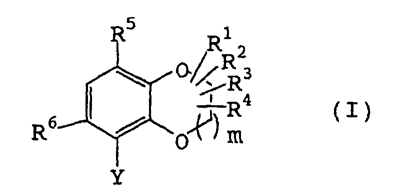 wherein m indicates an integer of 0 to 4;
R¹, R², R³ and R⁴ are the same or different, and each represents a hydrogen atom, substituted or unsubstituted lower alkyl, substituted or unsubstituted cycloalkyl, polycycloalkyl, substituted or unsubstituted lower alkoxycarbonyl, substituted or unsubstituted lower alkanoyl, substituted or unsubstituted lower alkanoyloxy, cyano, hydroxy, substituted or unsubstituted lower alkoxy, substituted or unsubstituted lower alkenyl, substituted or unsubstituted cycloalkenyl, substituted or unsubstituted aryl, a substituted or unsubstituted aromatic heterocyclic group, substituted or unsubstituted aralkyl or -CONR⁷R⁸ (wherein R⁷ and R⁸ are the same or different, and each represents a hydrogen atom, substituted or unsubstituted lower alkyl, substituted or unsubstituted lower alkanoyl, substituted or unsubstituted cycloalkyl, substituted or unsubstituted aryl, a substituted or unsubstituted aromatic heterocyclic group or substituted or unsubstituted aralkyl, or R⁷ and R⁸ are combined together with the adjacent nitrogen atom to form a substituted or unsubstituted heterocyclic group); two groups present on the same carbon atom among R¹, R², R³ and R⁴ are combined to represent a saturated spiro carbon ring together with the said carbon atom; two groups present on the adjacent carbon atoms among R¹, R², R³ and R⁴ are combined to represent a saturated carbon ring together with the said adjacent two carbon atoms; or two groups present on the adjacent carbon atoms among R¹, R², R³ and R⁴ are combined to represent a single bond (forming a double bond together with the already-existing bond);
R⁵ represents hydroxy or substituted or unsubstituted lower alkoxy;
R⁶ represents a hydrogen atom or halogen; and
Y represents the following formula (i): 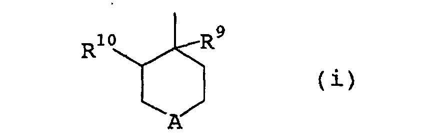 wherein A represents -CR¹¹R¹² (wherein R¹¹ and R¹² are the same or different, and each represents a hydrogen atom, hydroxy, formyl, cyano, substituted or unsubstituted lower alkoxy, a substituted or unsubstituted aromatic heterocyclic group, -NR¹³R¹⁴ (wherein R¹³ and R¹⁴ are the same or different, and each represents a hydrogen atom, substituted or unsubstituted lower alkyl, substituted or unsubstituted lower alkanoyl, substituted or unsubstituted cycloalkyl, substituted or unsubstituted aryl, a substituted or unsubstituted aromatic heterocyclic group, substituted or unsubstituted aralkyl or -SO₂R¹⁵ (wherein R¹⁵ represents lower alkyl, substituted or unsubstituted aryl or a substituted or unsubstituted aromatic heterocyclic group), or R¹³ and R¹⁴ are combined together with the adjacent nitrogen atom to form a substituted or unsubstituted heterocyclic group), -COOR¹⁶ (wherein R¹⁶ represents a hydrogen atom or substituted or unsubstituted lower alkyl), -CONR^{13a}R^{14a} (wherein R^{13a} and R^{14a} each have the same meaning as the above-described R¹³ and R¹⁴, respectively), -CONHOH or -CH₂COOR¹⁷ (wherein R¹⁷ represents a hydrogen atom or substituted or unsubstituted lower alkyl), or R¹¹ and R¹² are combined together to represent -OCH₂(CH₂)ₙO-(wherein n indicates an integer of 1 to 3), -CR¹⁸R¹⁹O- (wherein R¹⁸ and R¹⁹ are the same or different, and each represents a hydrogen atom or cyano), =CHOR²⁰ (wherein R²⁰ represents substituted or unsubstituted lower alkyl, substituted or unsubstituted lower alkenyl or substituted or unsubstituted aralkyl), =CHCOOR²¹ (wherein R²¹ represents a hydrogen atom or substituted or unsubstituted lower alkyl), =NOR²² (wherein R²² represents a hydrogen atom, substituted or unsubstituted lower alkyl, substituted or unsubstituted lower alkanoyl, substituted or unsubstituted cycloalkyl or substituted or unsubstituted aralkyl) or =O), -O-, -S(O)ₚ- (wherein p indicates an integer of 0 to 2), -COO- or -CONH-; R⁹ represents substituted lower alkyl, substituted lower alkoxy, substituted lower alkenyl, substituted or unsubstituted lower alkanoyl, substituted cycloalkyl, substituted cycloalkenyl, substituted or unsubstituted cycloalkanoyl, substituted or unsubstituted lower alkoxycarbonyl, substituted or unsubstituted aryl, a substituted or unsubstituted aromatic heterocyclic group, substituted or unsubstituted aralkyl, substituted or unsubstituted aroyl, substituted or unsubstituted heteroaroyl, -CH₂NR²³R²⁴ (wherein R²³ and R²⁴ are the same or different, and each represents a hydrogen atom, substituted or unsubstituted lower alkyl, substituted or unsubstituted lower alkanoyl, substituted or unsubstituted cycloalkyl, substituted or unsubstituted aryl, a substituted or unsubstituted aromatic heterocyclic group, substituted or unsubstituted aralkyl or -SO₂R²⁵ (wherein R²⁵ represents lower alkyl, substituted or unsubstituted aryl or a substituted or a unsubstituted aromatic heterocyclic group), or R²³ and R²⁴ are combined together with the adjacent nitrogen atom to form a substituted or unsubstituted heterocyclic group), formyl, -C≡C-C-R²⁶ (wherein R²⁶ represents halogen, carboxy, substituted or unsubstituted lower alkoxycarbonyl, substituted or unsubstituted lower alkyl, substituted or unsubstituted lower alkanoyl, substituted or unsubstituted cycloalkyl, substituted or unsubstituted aryl, a substituted or unsubstituted aromatic heterocyclic group or substituted or unsubstituted aralkyl), -(CH₂)_{q}CONR²⁷R²⁸ (wherein q indicates an integer of 1 to 3; and R²⁷ and R²⁸ are the same or different, and each represents a hydrogen atom, substituted or unsubstituted lower alkyl, substituted or unsubstituted cycloalkyl, substituted or unsubstituted aryl, a substituted or unsubstituted aromatic heterocyclic group or substituted or unsubstituted aralkyl, or R²⁷ and R²⁸ are combined together with the adjacent nitrogen atom to form a substituted or unsubstituted heterocyclic group), -CH=CHCONR^{27a}R^{28a} (wherein R^{27a} and R^{28a} each have the same meaning as the above-defined R²⁷ and R²⁸, respectively) or -CONR²⁹R³⁰ (wherein R²⁹ and R³⁰ are the same or different, and each represents a hydrogen atom, substituted or unsubstituted lower alkyl, substituted or unsubstituted cycloalkyl, substituted or unsubstituted aryl, a substituted or unsubstituted aromatic heterocyclic group or substituted or unsubstituted aralkyl, or R²⁹ and R³⁰ are combined together with the adjacent nitrogen atom to form a substituted or unsubstituted heterocyclic group, provided that R²⁹ and R³⁰ do not simultaneously represent a hydrogen atom); R¹⁰ represents a hydrogen atom; and in the case that R¹¹ and R¹² both are a hydrogen atom at the same time, in the case that one of R¹¹ and R¹² represents cyano, -NHSO₂R¹⁵ (wherein R¹⁵ has the same meaning as defined above), -CONHOH or a substituted or unsubstituted aromatic heterocyclic group other than substituted or unsubstituted tetrazolyl among the above definition, in the case that one of R¹¹ and R¹² represents a group other than cyano, -NHSO₂R¹⁵ (wherein R¹⁵ has the same meaning as defined above), -CONHOH or a substituted or unsubstituted aromatic heterocyclic group other than substituted or unsubstituted tetrazolyl among the above definition, and the other represents a hydrogen atom or a group other than substituted or unsubstituted lower alkoxy among the above definition, or in the case that R¹¹ and R¹² are combined together to represent =NOR²² (wherein R²² has the same meaning as defined above), then R⁹ may represent cyano, ethynyl or carbamoyl, or R⁹ and R¹⁰ may be combined to represent a single bond (forming a double bond together with the already-existing bond); in the case that R¹¹ and R¹² are the same or different and do not represent a hydrogen atom, hydroxy, lower alkoxy or carboxy among the above definition, R⁹ may represent a hydrogen atom, hydroxy, lower alkyl, lower alkoxy, lower alkenyl, cycloalkyl, halogen, cycloalkenyl or carboxy, or the following formula (ii): 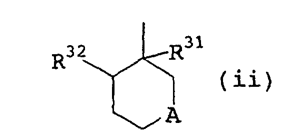 wherein A has the same meaning as defined above; R³¹ represents cyano, ethynyl, carbamoyl, substituted lower alkyl, substituted lower alkoxy, substituted lower alkenyl, substituted or unsubstituted lower alkanoyl, substituted cycloalkyl, substituted cycloalkenyl, substituted or unsubstituted cycloalkanoyl, substituted or unsubstituted lower alkoxycarbonyl, substituted or unsubstituted aryl, a substituted or unsubstituted aromatic heterocyclic group, substituted or unsubstituted aralkyl, substituted or unsubstituted aroyl, substituted or unsubstituted heteroaroyl, -CH₂NR^{23a}R^{24a} (wherein R^{23a} and R^{24a} each have the same meaning as the above-defined R²³ and R²⁴, respectively), formyl, -C≡C-R^{26a} (wherein R^{26a} has the same meaning as the above-defined R²⁶), -(CH₂)_{qa}CONR^{27b}R^{28b} (wherein qa, R^{27b} and R^{28b} each have the same meaning as the above-defined q, R²⁷ and R²⁸, respectively), -CH=CHCONR^{27c}R^{28c} (wherein R^{27c} and R^{28c} each have the same meaning as the above-defined R²⁷ and R²⁸, respectively) or -CONR^{27d}R^{28d} (wherein R^{27d} and R^{28d} each have the same meaning as the above-defined R²⁷ and R²⁸, respectively); R³² represents a hydrogen atom, or it is combined together with R³¹ to represent a single bond (forming a double bond together with the already-existing bond), and in the case that R¹¹ is not a hydrogen atom, hydroxy, lower alkoxy or carboxy among the above definition, R³¹ may represent a hydrogen atom, hydroxy, lower alkyl, lower alkoxy, lower alkenyl, cycloalkyl, halogen, cycloalkenyl or carboxy),
or a pharmaceutically acceptable salt thereof.

2. The oxygen-containing heterocyclic compound or a pharmaceutically acceptable salt thereof according to claim 1, wherein R⁹ is substituted lower alkyl, substituted lower alkenyl, substituted or unsubstituted cycloalkyl, substituted or unsubstituted cycloalkenyl, formyl, -C≡C-R²⁶ (wherein R²⁶ has the same meaning as defined above), -(CH₂)_{q}CONR²⁷R²⁸ (wherein q, R²⁷ and R²⁸ each have the same meaning as defined above) or -CH=CHCONR^{27a}R^{28a} (wherein R^{27a} and R^{28a} each have the same meaning as defined above), or R³¹ is substituted lower alkyl, substituted lower alkenyl, substituted or unsubstituted cycloalkyl, substituted or unsubstituted cycloalkenyl, formyl, -C≡C-R^{26a} (wherein R^{26a} has the same meaning as defined above), -(CH₂)_{qa}CONR^{27b}R^{28b} (wherein qa, R^{27b} and R^{28b} each have the same meaning as defined above) or -CH=CHCONR^{27c}R^{28c} (wherein R^{27c} and R^{28c} each have the same meaning as defined above).

3. The oxygen-containing heterocyclic compound or a pharmaceutically acceptable salt thereof according to claim 1 or 2, wherein R¹¹ is cyano or -COOR¹⁶ (wherein R¹⁶ has the same meaning as defined above).

4. A pharmaceutical composition comprising as an effective component the oxygen-containing heterocyclic compound or a pharmaceutically acceptable salt thereof according to any one of claims 1 to 3.

5. A phosphodiesterase IV inhibitor comprising as an effective component the oxygen-containing heterocyclic compound or a pharmaceutically acceptable salt thereof according to any one of claims 1 to 3.

6. Use of the oxygen-containing heterocyclic compound or a pharmaceutically acceptable salt thereof according to any one of claims 1 to 3 for the preparation of a phosphodiesterase IV inhibitor.

7. A method for inhibiting phosphodiesterase IV which comprises administering an effective amount of the oxygen-containing heterocyclic compound or a pharmaceutically acceptable salt thereof according to any one of claims 1 to 3.
